# EUROPEAN PATENT APPLICATION

(11) **EP 1 754 717 A1**
(43) Date of publication of application: **21.02.2007**
(21) Application number: 05018052.0
(22) Date of filing: 19.08.2005
(51) Int. Cl.: C07K 14/445, C07K 16/20, C12N 15/09, A61K 39/015, A61K 39/395, A61K 38/00

(54) **Antigenic peptides and their use**

(71) Applicant: Université de Lausanne, 1015 Lausanne (CH)
(72) Inventor: Corradin, Giampietro, c/o Institut de Biochimie, 1066 Epalinges (CH); Kajava, Andrey Dr., c/o CRBM Bioch. Macromoléc., 34293 Montpellier Cedex 5 (FR)
(74) Representative: Jelsch, Emmanuel Edwin

(57) **Abstract**

This invention relates generally to the field of pathogen peptidic antigens and their use, for example, for the preparation of a vaccine against said pathogen. More specifically, the present invention relates to an antigenic peptide deriving from plasmodium species sequences, and includes antibodies and methods of producing and using same.

### Annex to the application documents - subsequently filed sequences listing

SEQUENCE LISTING

<110>  Université de Lausanne

<120>  Antigenic peptides and their use

<130>  15173/EP

<140>  EP05018052
<141>  2005-08-19

<160>  133

<170>  PatentIn version 3.3

<210>  1
<211>  63
<212>  PRT
<213>  Plasmodium falciparum

<400>  1

Val Asn Asn Leu Asp Ser Thr Val Asn Tyr Met Asn Ser Thr Gly Asn
1               5                   10                  15

Asn Ile Asn Asn Ile Val Asn Asn Leu Asp Ser Thr Val Asn Tyr Met
            20                  25                  30

Asn Ser Thr Gly Asn Asn Ile Asn Asn Ile Val Asn Asn Leu Asp Ser
        35                  40                  45

Thr Val Asn Tyr Met Asn Ser Thr Gly Asn Asn Ile Asn Asn Ile
    50                  55                  60

<210>  2
<211>  27
<212>  PRT
<213>  Plasmodium falciparum

<400>  2

Thr Ile Ser Ser Leu Ser Asn Lys Ile Val Asn Tyr Glu Ser Lys Ile
1               5                   10                  15

Glu Glu Leu Glu Lys Glu Leu Lys Glu Val Lys
            20                  25

<210>  3
<211>  27
<212>  PRT
<213>  Plasmodium falciparum

<400>  3

Asn Ile Lys Thr Met Asn Thr Gln Ile Ser Thr Leu Lys Asn Asp Val
1               5                   10                  15

His Leu Leu Asn Glu Gln Ile Asp Lys Leu Asn
            20                  25

<210>  4
<211>  27
<212>  PRT
<213>  Plasmodium falciparum

<400>  4

Glu Ile Glu Lys Leu Asn Lys Gln Leu Thr Lys Cys Asn Lys Gln Ile
1               5                   10                  15

Asp Glu Leu Asn Glu Glu Val Glu Lys Leu Asn
            20                  25

<210>  5
<211>  25
<212>  PRT
<213>  Plasmodium falciparum

<400>  5

Ile Ile Asp Ile Lys Lys His Leu Glu Lys Leu Lys Ile Glu Ile Lys
1               5                   10                  15

Glu Lys Lys Glu Asp Leu Glu Asn Leu
            20                  25

<210>  6
<211>  25
<212>  PRT
<213>  Plasmodium falciparum

<400>  6

Ile Asn Asn Leu Asn Glu Lys Leu Glu Glu Thr Asn Lys Glu Tyr Thr
1               5                   10                  15

Asn Leu Gln Asn Asn Tyr Thr Asn Glu
            20                  25

<210>  7
<211>  25
<212>  PRT
<213>  Plasmodium falciparum

<400>  7

Ile Asp Lys Leu Asn Asn Glu Lys Gly Thr Leu Asn Ser Lys Ile Ser
1               5                   10                  15

Glu Leu Asn Val Gln Ile Met Asp Leu
            20                  25

<210>  8
<211>  46
<212>  PRT
<213>  Plasmodium falciparum

<400>  8

Ile Lys Thr Met Asn Thr Gln Ile Ser Thr Leu Lys Asn Asp Val His
1               5                   10                  15

Leu Leu Asn Glu Gln Ile Asp Lys Leu Asn Asn Glu Lys Gly Thr Leu
            20                  25                  30

Asn Ser Lys Ile Ser Glu Leu Asn Val Gln Ile Met Asp Leu
        35                  40                  45

<210>  9
<211>  25
<212>  PRT
<213>  Plasmodium falciparum

<400>  9

Leu Leu Ser Lys Asp Lys Glu Ile Glu Glu Lys Asn Lys Lys Ile Lys
1               5                   10                  15

Glu Leu Asn Asn Asp Ile Lys Lys Leu
            20                  25

<210>  10
<211>  25
<212>  PRT
<213>  Plasmodium falciparum

<400>  10

Leu Asn Leu Val Asp Gln Gly Lys Lys Lys Leu Lys Lys Asp Val Glu
1               5                   10                  15

Lys Gln Lys Lys Glu Ile Glu Lys Leu
            20                  25

<210>  11
<211>  49
<212>  PRT
<213>  Plasmodium falciparum

<400>  11

Ile Cys Ser Leu Thr Thr Glu Val Met Glu Leu Asn Asn Lys Lys Asn
1               5                   10                  15

Glu Leu Ile Glu Glu Asn Asn Lys Leu Asn Leu Val Asp Gln Gly Lys

20                  25                  30

Lys Lys Leu Lys Lys Asp Val Glu Lys Gln Lys Lys Glu Ile Glu Lys
        35                  40                  45

Leu

<210>  12
<211>  77
<212>  PRT
<213>  Plasmodium falciparum

<400>  12

Val Asp Lys Ile Glu Glu His Ile Leu Asp Tyr Asp Glu Glu Ile Asn
1               5                   10                  15

Lys Ser Arg Ser Asn Leu Phe Gln Leu Lys Asn Glu Ile Cys Ser Leu
            20                  25                  30

Thr Thr Glu Val Met Glu Leu Asn Asn Lys Lys Asn Glu Leu Ile Glu
        35                  40                  45

Glu Asn Asn Lys Leu Asn Leu Val Asp Gln Gly Lys Lys Lys Leu Lys
    50                  55                  60

Lys Asp Val Glu Lys Gln Lys Lys Glu Ile Glu Lys Leu
65                  70                  75

<210>  13
<211>  25
<212>  PRT
<213>  Plasmodium falciparum

<400>  13

Leu Asp Glu Asn Glu Asp Asn Ile Lys Lys Met Lys Ser Lys Ile Asp
1               5                   10                  15

Asp Met Glu Lys Glu Ile Lys Tyr Arg
            20                  25

<210>  14
<211>  41
<212>  PRT
<213>  Plasmodium falciparum

<400>  14

Gly Met Asn Asn Met Asn Gly Asp Ile Asn Asn Ile Asn Gly Asp Ile
1               5                   10                  15

Asn Asn Met Asn Gly Asp Ile Asn Asn Met Asn Gly Asp Ile Asn Asn
            20                  25                  30

Met Asn Gly Asp Ile Asn Asn Met Asn
        35                  40

<210>  15
<211>  27
<212>  PRT
<213>  Plasmodium falciparum

<400>  15

Leu Ile Lys Tyr Met Asn Glu Arg Tyr Gln Asn Met Gln Gln Gly Tyr
1               5                   10                  15

Asn Asn Leu Thr Asn Tyr Ile Asn Gln Tyr Glu
            20                  25

<210>  16
<211>  27
<212>  PRT
<213>  Plasmodium falciparum

<400>  16

Asp Ile Asn Ser Thr Asn Asn Asn Leu Asp Asn Met Leu Ser Glu Ile
1               5                   10                  15

Asn Ser Ile Gln Asn Asn Ile His Thr Tyr Ile
            20                  25

<210>  17
<211>  22
<212>  PRT
<213>  Plasmodium falciparum

<400>  17

Glu Lys Lys Leu Asp Ile Leu Lys Val Asn Ile Ser Asn Ile Asn Asn
1               5                   10                  15

Ser Leu Asp Lys Leu Lys
            20

<210>  18
<211>  27
<212>  PRT
<213>  Plasmodium falciparum

<400>  18

Asp Val His Asn Ile Lys Glu Asp Tyr Asn Leu Leu Gln Gln Tyr Leu
1               5                   10                  15

Asn Tyr Met Lys Asn Glu Met Glu Gln Leu Lys
            20                  25

<210>  19
<211>  25
<212>  PRT
<213>  Plasmodium falciparum

<400>  19

Asp Glu Lys Ile Asn Asp Tyr Leu Glu Glu Ile Lys Asn Glu Gln Asn
1               5                   10                  15

Lys Ile Asp Lys Thr Ile Asp Asp Ile
            20                  25

<210>  20
<211>  25
<212>  PRT
<213>  Plasmodium falciparum

<400>  20

Met Asp Val Val Ile Asn Gln Leu Arg Asp Ile Asp Arg Gln Met Leu
1               5                   10                  15

Asp Leu Tyr Lys Glu Leu Asp Glu Lys
            20                  25

<210>  21
<211>  25
<212>  PRT
<213>  Plasmodium falciparum

<400>  21

Ile Ser Asn Ile Phe Lys Asp Ile Gln Asn Ile Lys Lys Gln Ser Gln
1               5                   10                  15

Asp Ile Ile Thr Asn Met Asn Asp Met
            20                  25

<210>  22
<211>  25
<212>  PRT
<213>  Plasmodium falciparum

<400>  22

Leu Glu Glu Ile Ile Lys Asn Leu Asp Ile Leu Asp Glu Gln Ile Met
1               5                   10                  15

Thr Tyr His Asn Ser Ile Asp Glu Leu

20                    25

<210>  23
<211>  25
<212>  PRT
<213>  Plasmodium falciparum

<400>  23

Phe Gln Lys Val Lys Glu Lys Ala Glu Ile Gln Lys Glu Asn Ile Glu
1               5                   10                  15

Lys Ile Lys Gln Glu Ile Asn Thr Leu
            20                  25

<210>  24
<211>  54
<212>  PRT
<213>  Plasmodium falciparum

<400>  24

Glu Lys Lys Leu Asp Ile Leu Lys Val Asn Ile Ser Asn Ile Asn Asn
1               5                   10                  15

Ser Leu Asp Lys Leu Lys Lys Tyr Tyr Glu Glu Ala Leu Phe Gln Lys
            20                  25                  30

Val Lys Glu Lys Ala Glu Ile Gln Lys Glu Asn Ile Glu Lys Ile Lys
        35                  40                  45

Gln Glu Ile Asn Thr Leu
    50

<210>  25
<211>  34
<212>  PRT
<213>  Plasmodium falciparum

<400>  25

Asp Val Thr His Leu Thr Asn Asp Val Thr His Leu Thr Asn Asp Val
1               5                   10                  15

Thr His Leu Thr Asn Asp Val Thr His Leu Thr Asn Asp Val Thr His
            20                  25                  30

Leu Thr

<210>  26
<211>  27
<212>  PRT

<213>  Plasmodium falciparum

<400>  26

Asn Leu Asn Lys Val Lys Ile Asn Ile Asn Asp Leu Asn Asn Asn Ile
1               5                   10                  15

Val Asp Val Asn Asn Ser Ile His Asn Ile Glu
            20                  25

<210>  27
<211>  27
<212>  PRT
<213>  Plasmodium falciparum

<400>  27

Lys Lys Arg Asn Val Glu Glu Glu Leu His Ser Leu Arg Lys Asn Tyr
1               5                   10                  15

Asn Ile Ile Asn Glu Glu Ile Glu Glu Ile Thr
            20                  25

<210>  28
<211>  25
<212>  PRT
<213>  Plasmodium falciparum

<400>  28

Tyr Ile Asp Ile Lys Lys Lys Ile Ser Glu Leu Gln Lys Asp Asn Glu
1               5                   10                  15

Ser Leu Lys Ile Gln Val Asp Arg Leu
            20                  25

<210>  29
<211>  27
<212>  PRT
<213>  Plasmodium falciparum

<400>  29

Gln Met Glu Gly Phe Gln Lys Gln Leu Asp Arg Leu Ser Asp Ser Leu
1               5                   10                  15

Ser Lys Ile Gln Lys Ala Leu Gly Glu Tyr Leu
            20                  25

<210>  30
<211>  25
<212>  PRT
<213>  Plasmodium falciparum

<400>  30

Leu Lys Leu Asn Glu Gly Leu Glu Asn Ile Lys Gln Glu Leu His Ile
1               5                   10                  15

Ile Asp Arg Glu Leu Lys Asn Ile Leu
            20                  25

<210>  31
<211>  27
<212>  PRT
<213>  Plasmodium falciparum

<400>  31

Asn Ile Asn Ser Val Asn Asn Asn Ile Asn Ser Val Asp Asn Asn Ile
1               5                   10                  15

Asn Asn Val Asp Asn Asn Ile Asn Ser Val Asn
            20                  25

<210>  32
<211>  27
<212>  PRT
<213>  Plasmodium falciparum

<400>  32

Asn Ile Ile Gln Ile Lys Asn Asp Ile Glu Gln Cys Gln Lys Ser Ile
1               5                   10                  15

Lys Lys Ile Glu Asp Asn Leu Asn Thr Tyr Glu
            20                  25

<210>  33
<211>  27
<212>  PRT
<213>  Plasmodium falciparum

<400>  33

Tyr Ile Asp Asp Val Asp Arg Asp Val Glu Asn Tyr Asp Lys Gly Ile
1               5                   10                  15

Ala Asn Val Asp His His Leu Asn Asp Val His
            20                  25

<210>  34
<211>  27
<212>  PRT
<213>  Plasmodium falciparum

<400>  34

Asn Met Asn Asn Met Asn Asn Asn Met Asn Asn Met Asn Asn Asn Met
1               5                   10                  15

Asn Asn Asn Met Asn Asn Met Asn Asn Met Asn
            20                  25

<210>  35
<211>  27
<212>  PRT
<213>  Plasmodium falciparum

<400>  35

Ala Arg Asp Asp Ile Gln Lys Asp Ile Asn Lys Met Glu Ser Glu Leu
1               5                   10                  15

Ile Asn Val Ser Asn Glu Ile Asn Arg Leu Asp
            20                  25

<210>  36
<211>  27
<212>  PRT
<213>  Plasmodium falciparum

<400>  36

Ser Ser Asn Asn Leu Ser Asp Gln Ile Asn Ile Leu Asn Asn Asn Ile
1               5                   10                  15

Gln His Ile Asn Ser Thr Phe Asn Asn Leu Arg
            20                  25

<210>  37
<211>  27
<212>  PRT
<213>  Plasmodium falciparum

<400>  37

Asn Asn Asn Val Asn Asn Ile Asn Met Asn Asn Ile Asn Ser Asn Val
1               5                   10                  15

Asn Asn Ile Asn Asn Ser Met Asn Asn Ile Asn
            20                  25

<210>  38
<211>  27
<212>  PRT
<213>  Plasmodium falciparum

<400>  38

Asn Ile Thr Asn Ile Asn Lys Asn Ile Glu Asn Ile Lys Asn Asp Met
1               5                   10                  15

Ser Asn Leu Asn Asn Met Asn Asp Ser Asn Gln

20                  25

<210>  39
<211>  36
<212>  PRT
<213>  Plasmodium falciparum

<400>  39

Asn His Asp Thr Arg Ile Asn Asp Tyr Asn Lys Arg Leu Thr Glu Tyr
1               5                   10                  15

Asn Lys Arg Leu Thr Glu Tyr Asn Lys Arg Leu Thr Glu Tyr Thr Lys
            20                  25                  30

Arg Leu Asn Glu
        35

<210>  40
<211>  25
<212>  PRT
<213>  Plasmodium falciparum

<400>  40

Met Glu Gln Met Asp Met Lys Met Glu Lys Ile Asp Val Asn Met Asp
1               5                   10                  15

Gln Met Asp Val Lys Met Glu Gln Met
            20                  25

<210>  41
<211>  30
<212>  PRT
<213>  Plasmodium falciparum

<400>  41

Asn Asn Val Ile Arg Ser Lys Met Tyr Asn Ile Lys Lys Arg Ile Ser
1               5                   10                  15

Lys Ile Asn Asp Glu Leu His Glu Leu Ser Asn Phe Phe Leu
            20                  25                  30

<210>  42
<211>  25
<212>  PRT
<213>  Plasmodium falciparum

<400>  42

Leu Glu Glu Met Lys Gln Lys Asn Lys Glu Leu Ile Asn Asn Leu Asn
1               5                   10                  15

Asp Ile Ser Asp Glu Leu Lys Asn Cys
            20                  25

<210>  43
<211>  29
<212>  PRT
<213>  Plasmodium falciparum

<400>  43

Glu Lys Leu Asn Asp Met Gln Lys Lys Leu Asn Asp Val Asn Glu Lys
1               5                   10                  15

Tyr Lys Asn Ile Val Glu Cys Leu Asn Asn Tyr Lys Thr
            20                  25

<210>  44
<211>  25
<212>  PRT
<213>  Plasmodium falciparum

<400>  44

Leu Lys Glu Lys Asp Lys Lys Ile Asn Asp Leu Met Lys Asp Met Glu
1               5                   10                  15

Lys Lys Lys Glu Glu Ile Asn Lys Leu
            20                  25

<210>  45
<211>  40
<212>  PRT
<213>  Plasmodium falciparum

<400>  45

Asn Glu Ile Lys Glu Leu Asn Asn Thr Leu Asn Lys Tyr Lys Glu Glu
1               5                   10                  15

Met Asn Asn Tyr Lys Glu Glu Ile Ile Val Ile Asn Glu Lys Tyr Lys
            20                  25                  30

Leu Leu Glu Ile Glu Leu Cys Lys
        35                  40

<210>  46
<211>  25
<212>  PRT
<213>  Plasmodium falciparum

<400>  46

Met Asn Ile Met Glu Asn Asp Met Asn Ile Met Glu Asn Asp Met Asn
1               5                   10                  15

Ile Ile Lys Asn Asp Met Asn Ile Met
            20                  25

<210>  47
<211>  25
<212>  PRT
<213>  Plasmodium falciparum

<400>  47

Met Asn Ile Met Glu Lys Asp Met Asn Ile Ile Lys Asn Asp Met Asn
1               5                   10                  15

Ile Ile Lys Asn Asn Met Asn Ile Ile
            20                  25

<210>  48
<211>  29
<212>  PRT
<213>  Plasmodium falciparum

<400>  48

Glu Glu Ile Tyr Lys Leu Asn Asn Asp Ile Asp Met Leu Ser Asn Asn
1               5                   10                  15

Cys Lys Lys Leu Lys Glu Ser Ile Met Met Met Glu Lys
            20                  25

<210>  49
<211>  29
<212>  PRT
<213>  Plasmodium falciparum

<400>  49

Lys Glu Ile Gln Met Leu Lys Asn Gln Ile Leu Ser Leu Glu Glu Ser
1               5                   10                  15

Ile Lys Ser Leu Asn Glu Phe Ile Asn Asn Leu Lys Asn
            20                  25

<210>  50
<211>  31
<212>  PRT
<213>  Plasmodium falciparum

<400>  50

Lys Asn Val Ile Glu Leu Lys Glu Tyr Leu Glu Asp Leu Lys Lys Arg
1               5                   10                  15

Met Phe Asp Met Gln Lys Arg Leu Asn Asp Ile Ile Ile Thr Lys
            20                  25                  30

<210>  51
<211>  25
<212>  PRT
<213>  Plasmodium falciparum

<400>  51

Ile Glu Thr Leu Glu Asn Asn Leu Lys Ile Gly Lys Glu Lys Ile Asn
1               5                   10                  15

Lys Phe Asp Asn Glu Ile Gln Lys Leu
            20                  25

<210>  52
<211>  25
<212>  PRT
<213>  Plasmodium falciparum

<400>  52

Ile Asn Asn Met Lys Glu Gln Ile Glu Asp Val Asn His Lys Ile Ala
1               5                   10                  15

Ser Ile Asn Lys Glu Lys Glu Glu Leu
            20                  25

<210>  53
<211>  25
<212>  PRT
<213>  Plasmodium falciparum

<400>  53

Leu Lys Glu Leu Glu Lys Lys Asn Asp Ile Leu Ser Asn Asp Lys Asn
1               5                   10                  15

Gln Leu Gln Lys Asn Leu Thr Gln Leu
            20                  25

<210>  54
<211>  25
<212>  PRT
<213>  Plasmodium falciparum

<400>  54

Leu Ser Glu Gly Asn Lys Glu Leu Glu Lys Leu Glu Lys Asn Ile Lys
1               5                   10                  15

Glu Leu Glu Glu Thr Asn Asn Thr Leu
            20                  25

<210>  55

<211>  25
<212>  PRT
<213>  Plasmodium falciparum

<400>  55

Leu Asn His Leu Thr Asn Asn Leu Ser His His Asn Asn His Met Asn
1               5                   10                  15

His His Thr Ser Asn Leu Asn His Ile
            20                  25

<210>  56
<211>  38
<212>  PRT
<213>  Plasmodium falciparum

<400>  56

Ser Thr Asp Ile Asn Ser Leu Asn Asp Glu Val Lys Lys Leu Lys Glu
1               5                   10                  15

Glu Leu Asn Lys Ile Arg Asn Glu Tyr Asp Asp Phe Lys Asn Lys Leu
            20                  25                  30

Glu Leu Leu Tyr Gln Lys
        35

<210>  57
<211>  25
<212>  PRT
<213>  Plasmodium falciparum

<400>  57

Ile Asn Asn Met Asp Glu Lys Ile Asn Asn Val Asp Glu Gln Asn Asn
1               5                   10                  15

Asn Met Asp Glu Lys Ile Asn Asn Val
            20                  25

<210>  58
<211>  34
<212>  PRT
<213>  Plasmodium falciparum

<400>  58

Asp Asn Asn Val Asn Asn Met Asp Asn Asn Val Asn Asn Val Asp Asn
1               5                   10                  15

Asn Val Asn Asn Val Asp Asn Asn Leu Asn Asn Val Asp Asn Asn Val
            20                  25                  30

Asn Asn

<210> 59
<211> 29
<212> PRT
<213> Plasmodium falciparum

<400> 59

Thr Leu Ile Asp Ser Phe Asn Leu Asn Leu Ser Tyr Leu Arg Glu Ser
1               5                   10                  15

Ile Asn Asn Lys Lys Lys His Ile Asn Lys Ile Asn Asp
            20                  25

<210> 60
<211> 25
<212> PRT
<213> Plasmodium falciparum

<400> 60

Leu Leu Asn Leu Gln Asn Asp Ile Gln Lys Lys Asn Lys Thr Leu Glu
1               5                   10                  15

Glu Leu Gly Glu Glu Ile Gln Lys Tyr
            20                  25

<210> 61
<211> 33
<212> PRT
<213> Plasmodium falciparum

<400> 61

Met Ser Gln Glu Lys Ile Ser Glu Ile Ile Lys Asp Ile Ser Ala Leu
1               5                   10                  15

Lys Thr Ser Cys Glu Lys Leu Asn Ser Gln Leu Asp Glu Leu Ile Thr
            20                  25                  30

Gln

<210> 62
<211> 25
<212> PRT
<213> Plasmodium falciparum

<400> 62

Phe Asn Asp Val Thr Ser Asn Val Gly Asn Met Asn Asn Asn Leu Thr
1               5                   10                  15

Asn Phe Ser Asn Pro Tyr Asn Ser Val
            20                  25

<210>  63
<211>  37
<212>  PRT
<213>  Plasmodium falciparum

<400>  63

Ile Pro Leu Asn Gln Lys Val Leu Glu Ile Ser Lys Lys Leu Asn Asn
1               5                   10                  15

Met Asn Asn Asn Ile Asn Glu Tyr Lys Asn Tyr Leu Ser Asn Phe Ile
            20                  25                  30

His Met Leu Lys Glu
        35

<210>  64
<211>  35
<212>  PRT
<213>  Plasmodium falciparum

<400>  64

Asn Asn Ile Asn Asn Ile Asn Asn Asn Ile Asn Asn Ile Asn Asn Asn
1               5                   10                  15

Ile Asn Asn Ile Asn Asn Asn Ile Asn Asn Ile Asn Asn Asn Val Asn
            20                  25                  30

Asn Tyr Tyr
        35

<210>  65
<211>  25
<212>  PRT
<213>  Plasmodium falciparum

<400>  65

Met Asn Asn Met Asn Asn Asn Met Asn Asn Leu Asn Asn Ser Ile Asn
1               5                   10                  15

Asn Asn Asn Met Asn Asn Ile Asn Val
            20                  25

<210>  66
<211>  25
<212>  PRT
<213>  Plasmodium falciparum

<400>  66

Leu Lys Lys Lys Asp Glu Glu Ile Lys His Leu Asn Glu Ser Ile Tyr
1               5                   10                  15

Ile Leu Gln Gln Asn Met Asn Asn Leu
            20                  25

<210>  67
<211>  25
<212>  PRT
<213>  Plasmodium falciparum

<400>  67

Phe Asp Ala Tyr Asn Glu Lys Leu Gly Ser Ile Ser Gln Ser Ile Asp
1               5                   10                  15

Glu Ile Lys Lys Lys Ile Asp Asn Leu
            20                  25

<210>  68
<211>  33
<212>  PRT
<213>  Plasmodium falciparum

<400>  68

Asp Ser Met Asn Asn His Lys Asp Asp Met Asn Asn Tyr Asn Asp Asn
1               5                   10                  15

Ile Asn Asn Tyr Val Glu Ser Met Asn Asn Tyr Asp Asp Ile Met Asn
            20                  25                  30

Lys

<210>  69
<211>  30
<212>  PRT
<213>  Plasmodium falciparum

<400>  69

Asn Asn Phe Val Asn Asn Lys Met Asn Asn Met Asn Asn Met Lys Asn
1               5                   10                  15

Asn Met Asn Asn Met Asn Asn Ile Met Asn Asn Ile Met Asn
            20                  25                  30

<210>  70
<211>  25
<212>  PRT
<213>  Plasmodium falciparum

<400>  70

Ile Asp Val Ile Lys Asn Lys His Asn Lys Leu Asn Glu Lys Ile Asn
1               5                   10                  15

Ser Leu Glu Glu Lys Leu Ile Glu Met
            20                  25

<210>  71
<211>  31
<212>  PRT
<213>  Plasmodium falciparum

<400>  71

Ser Asn Lys Thr Phe Glu Lys Leu Asn Glu Lys Leu Asn Asp Ile Arg
1               5                   10                  15

Asn Asp Val Thr Asn Tyr Lys Asn Glu Leu Glu Glu Phe Lys Asn
            20                  25                  30

<210>  72
<211>  39
<212>  PRT
<213>  Plasmodium falciparum

<400>  72

Asn Ser Leu Asp Tyr Tyr Lys Lys Val Ile Ile Lys Leu Lys Asn Asn
1               5                   10                  15

Ile Asn Asn Met Glu Glu Tyr Thr Asn Asn Ile Thr Asn Asp Ile Asn
            20                  25                  30

Val Leu Lys Ala His Ile Asp
        35

<210>  73
<211>  25
<212>  PRT
<213>  Plasmodium falciparum

<400>  73

Met Asp Thr Val Tyr Lys Asn Ile Ile Asn Met Ser Asn Asn Met Thr
1               5                   10                  15

Gln Met Tyr Asn Ser Met Asn Asn Met
            20                  25

<210>  74
<211>  25
<212>  PRT

<213>  Plasmodium falciparum

<400>  74

Val Asn Lys Met Asn Glu Glu Val Asn Lys Met Asn Glu Glu Val Asn
1               5                   10                  15

Lys Met Asn Glu Glu Val Asn Lys Met
            20                  25

<210>  75
<211>  25
<212>  PRT
<213>  Plasmodium falciparum

<400>  75

Val Asn Lys Met Asn Lys Glu Val Asn Lys Met Asp Glu Glu Val Asn
1               5                   10                  15

Lys Met Asn Lys Glu Val Asn Lys Met
            20                  25

<210>  76
<211>  25
<212>  PRT
<213>  Plasmodium falciparum

<400>  76

Leu Lys Ser Val Asp Asn Tyr Leu Glu Lys Ile Asn Asn Lys Ile Lys
1               5                   10                  15

Asp Leu Asp Asp Asn Ile Asn Asp Arg
            20                  25

<210>  77
<211>  25
<212>  PRT
<213>  Plasmodium falciparum

<400>  77

Ile Asn Asn Leu Glu Thr Asn Ile Asn Asp Tyr Asn Lys Lys Ile Lys
1               5                   10                  15

Glu Gly Asp Ser Gln Leu Asn Asn Ile
            20                  25

<210>  78
<211>  28
<212>  PRT
<213>  Plasmodium falciparum

<400>  78

Glu Lys Leu Lys Lys Tyr Asn Asn Glu Ile Ser Ser Leu Lys Lys Glu
1               5                   10                  15

Leu Asp Ile Leu Asn Glu Lys Met Gly Lys Cys Thr
            20                  25

<210>  79
<211>  25
<212>  PRT
<213>  Plasmodium falciparum

<400>  79

Phe Asp Asn Val Asn Glu Asn Phe Glu Ser Ile Asn Glu Pro Tyr Asp
1               5                   10                  15

Ser Ile Asn Glu Pro Tyr Asp Asn Ile
            20                  25

<210>  80
<211>  25
<212>  PRT
<213>  Plasmodium falciparum

<400>  80

Phe Asn Arg Leu Asn Lys Glu Leu Asn Glu Leu Arg Glu Arg Tyr Asp
1               5                   10                  15

His Ile Ile Lys His Lys Lys Gln Met
            20                  25

<210>  81
<211>  25
<212>  PRT
<213>  Plasmodium falciparum

<400>  81

Leu Gln Asp Leu Lys Lys Lys Lys Asn Leu Leu Gln Glu Glu Ile Thr
1               5                   10                  15

Asn Tyr Lys Glu Glu Ile Lys Thr Leu
            20                  25

<210>  82
<211>  25
<212>  PRT
<213>  Plasmodium falciparum

<400>  82

Leu Ile Ile Leu Asp Asn Lys Glu Lys Gln Asn Gln Gln Asn Ile Asn
1               5                   10                  15

His Leu Lys Glu Glu Ile Asn Ser Met
            20                  25

<210>  83
<211>  25
<212>  PRT
<213>  Plasmodium falciparum

<400>  83

Ile Asn Glu Glu Glu Asn Ser Leu Asn Glu Met Lys Glu Asp Ile Asn
1               5                   10                  15

Glu Tyr Val Glu Met Glu Asn Lys Leu
            20                  25

<210>  84
<211>  25
<212>  PRT
<213>  Plasmodium falciparum

<400>  84

Met Asn Lys Leu Lys Asp Glu Glu Lys Lys Ile Gln Glu Glu Glu Lys
1               5                   10                  15

Lys Leu Asn Glu Glu Leu Glu Asn Ile
            20                  25

<210>  85
<211>  25
<212>  PRT
<213>  Plasmodium falciparum

<400>  85

Leu Ser Gly Leu Gln Asn Ser Leu Ser Gly Leu Lys Thr Pro Leu Ser
1               5                   10                  15

Gly Leu Gln Asn Ser Leu Ser Gly Leu
            20                  25

<210>  86
<211>  37
<212>  PRT
<213>  Plasmodium falciparum

<400>  86

Glu Glu Ile Lys Glu Glu Ile Lys Glu Val Lys Glu Glu Ile Lys Glu
1               5                   10                  15

Val Lys Glu Glu Ile Lys Glu Val Lys Glu Glu Ile Lys Glu Val Lys

20                  25                  30

Glu Glu Ile Lys Glu
        35

<210>  87
<211>  41
<212>  PRT
<213>  Plasmodium falciparum

<400>  87

Ile Glu Ile Asn Met Leu Thr Asn Asn Leu Leu Arg Glu Met Met Lys
1               5                   10                  15

Ile Lys Asn Lys Leu Gln Lys Leu Ser Asn Leu Leu Asn Ala Leu Arg
            20                  25                  30

Ser Asn Ile Glu Lys Ile Leu Lys Asn
        35                  40

<210>  88
<211>  36
<212>  PRT
<213>  Plasmodium falciparum

<400>  88

Lys Gly Leu Glu Glu Ala Asn Glu Lys Leu Gln Ile Val Arg Glu Lys
1               5                   10                  15

Val Gln Ser Leu Lys Ala Lys Leu Ser Glu Leu Ile Ser Gln Tyr Asp
            20                  25                  30

His Ala Ile Tyr
        35

<210>  89
<211>  25
<212>  PRT
<213>  Plasmodium falciparum

<400>  89

Val Glu Thr Leu Glu Lys Lys Leu Lys Glu Ile Tyr Asn Asp Glu Gln
1               5                   10                  15

Asn Tyr Lys Asn Ser Leu Gln Asn Ile
            20                  25

<210>  90
<211>  25
<212>  PRT

<213>  Plasmodium falciparum

<400>  90

Ile Asp Lys Ile Asn Glu Asn Ile Asn Arg Ile Lys Asn Asn Ile Lys
1               5                   10                  15

Lys Leu Asn Asp Asp Ile Asn Glu Leu
            20                  25

<210>  91
<211>  25
<212>  PRT
<213>  Plasmodium falciparum

<400>  91

Leu Gln Lys Leu Glu Glu Asp Lys Ile Asn Ile Tyr Lys Asn Leu Asn
1               5                   10                  15

Asn Leu Asn Gln Glu Leu Asn Gln Leu
            20                  25

<210>  92
<211>  25
<212>  PRT
<213>  Plasmodium falciparum

<400>  92

Leu Glu Glu Lys Thr Lys Gln Tyr Asn Asp Leu Gln Asn Asn Met Lys
1               5                   10                  15

Thr Ile Lys Glu Gln Asn Glu His Leu
            20                  25

<210>  93
<211>  40
<212>  PRT
<213>  Plasmodium falciparum

<400>  93

Arg Leu Ile Asn Asn Ile Glu Glu Ile Tyr Asn Ser Asn Cys Glu Gln
1               5                   10                  15

Ile Gln Asn Val Arg Asp Glu Phe Ala Glu Leu Lys Asn Asp Leu Asn
            20                  25                  30

Lys Ile Met Asn Leu Ile Asn Ile
        35                  40

<210>  94
<211>  25

<212>  PRT
<213>  Plasmodium falciparum

<400>  94

Ile Lys Glu Ile Asn Lys Ser Leu Thr Glu Val Lys Asn Glu Leu Thr
1               5                   10                  15

Glu Leu Gln Lys Asn Gln Glu Glu Ala
            20                  25

<210>  95
<211>  31
<212>  PRT
<213>  Plasmodium falciparum

<400>  95

Ser Ser Ile Ser Ser Ser Leu Thr Asn Ile Ser Ser Ser Leu Thr Asn
1               5                   10                  15

Ile Ser Ser Ser Leu Thr Asn Ile Ser Ser Ser Leu Ser Asn Ser
            20                  25                  30

<210>  96
<211>  30
<212>  PRT
<213>  Plasmodium falciparum

<400>  96

Met Cys Glu Leu Asn Val Met Glu Asn Asn Met Asn Asn Ile His Ser
1               5                   10                  15

Asn Asn Asn Asn Ile Ser Thr His Met Asp Asp Val Ile Glu
            20                  25                  30

<210>  97
<211>  25
<212>  PRT
<213>  Plasmodium falciparum

<400>  97

Leu Lys Glu Tyr Ser Ser Lys Leu Gln Glu Arg Glu Lys Lys Leu Lys
1               5                   10                  15

Glu Lys Lys Asn Glu Leu Gln Lys Val
            20                  25

<210>  98
<211>  25
<212>  PRT
<213>  Plasmodium falciparum

<400>  98

Ile Asp Asn Ile Asn Lys Asn Ile Asn Cys Ile Asn Asn Asp Val Asp
1               5                   10                  15

Asn Ile Asn Ser Asn Ile Asn Asn Ile
            20                  25

<210>  99
<211>  34
<212>  PRT
<213>  Plasmodium falciparum

<400>  99

Lys Ile Gln Ile Glu Glu Ile Lys Lys Glu Thr Asn Gln Ile Asn Lys
1               5                   10                  15

Asp Ile Asp His Ile Glu Met Asn Ile Ile Asn Leu Lys Lys Lys Ile
            20                  25                  30

Glu Phe

<210>  100
<211>  25
<212>  PRT
<213>  Plasmodium falciparum

<400>  100

Ile Glu Ile Glu Arg Glu Lys Ile Asn Gln Leu Gln Glu Glu Ile Lys
1               5                   10                  15

Lys Leu Gln Asn Glu Lys Asn Asp Leu
            20                  25

<210>  101
<211>  30
<212>  PRT
<213>  Plasmodium falciparum

<400>  101

Thr Tyr Thr Leu Ser Lys Leu Asn Asn Gln Ile Asn Glu Leu Thr Lys
1               5                   10                  15

Lys Ile Asn Ile Leu Arg Gly Asn Leu Asp Lys Ala Arg Lys
            20                  25                  30

<210>  102
<211>  35
<212>  PRT
<213>  Plasmodium falciparum

<400>  102

Lys Asn Asp Ile Asn Val Gln Leu Asp Asp Ile Asn Val Gln Leu Asp
1               5                   10                  15

Asp Ile Asn Val Gln Leu Asp Asp Ile Asn Ile Gln Leu Asp Glu Ile
            20                  25                  30

Asn Leu Asn
        35

<210>  103
<211>  25
<212>  PRT
<213>  Plasmodium falciparum

<400>  103

Lys Glu Glu Ile Asn Glu Lys Leu Gln Leu Leu Asn Asn Asp Leu Asp
1               5                   10                  15

Lys Lys Asn Glu Glu Leu Asn Ile Leu
            20                  25

<210>  104
<211>  25
<212>  PRT
<213>  Plasmodium falciparum

<400>  104

Ile Lys Asn Leu Asn Asn Glu Ile Asn Thr Leu Asn Asp Met Leu Lys
1               5                   10                  15

Asp Ser Glu Glu Glu Ile Arg Met Leu
            20                  25

<210>  105
<211>  25
<212>  PRT
<213>  Plasmodium falciparum

<400>  105

Leu Asn His Ile Ile Asn Glu Leu Glu Ile Lys Arg Glu Glu Ile Asn
1               5                   10                  15

Gln Met Asn Asn Lys Leu Asn Glu Leu
            20                  25

<210>  106
<211>  25
<212>  PRT

<213>  Plasmodium falciparum

<400>  106

Val Lys Ile Leu Lys Lys Arg Leu Asn Ser Ile Ser Asn Asp Leu Glu
1               5                   10                  15

Lys Arg Thr Glu Glu Ile Glu His Leu
            20                  25

<210>  107
<211>  25
<212>  PRT
<213>  Plasmodium falciparum

<400>  107

Ile Asn Val Leu Asn Asp Glu Ile Thr Lys Leu Lys Asn Glu Ile Asn
1               5                   10                  15

Thr Tyr Lys Asn Asp Leu Lys Asn Ile
            20                  25

<210>  108
<211>  25
<212>  PRT
<213>  Plasmodium falciparum

<400>  108

Ile Asn Lys Leu Asn Gln Gln Ile Asn Tyr Leu Gln Asp Asp Ile Asn
1               5                   10                  15

Ser Lys Ser Asp Asn Ile Ile Ser Leu
            20                  25

<210>  109
<211>  30
<212>  PRT
<213>  Plasmodium falciparum

<400>  109

Asn Phe Phe Leu Glu Gln Met Glu Asn Asp Met Ser Ser Thr Tyr Asp
1               5                   10                  15

Lys Met Asn Arg Ile Asn Met Asp Leu Ser Lys Leu Lys Arg
            20                  25                  30

<210>  110
<211>  29
<212>  PRT
<213>  Plasmodium falciparum

<400>  110

Glu Lys Leu Tyr Ile Leu Glu Lys Ser Ile Asn Lys Leu Lys Lys Leu
1               5                   10                  15

Leu Asn Asp Ile Asn Asn Lys Tyr Gln Thr Ile Lys Lys
            20                  25

<210>  111
<211>  25
<212>  PRT
<213>  Plasmodium falciparum

<400>  111

Leu Asn Gln Val Leu Glu Lys Tyr Glu Glu Leu Lys Lys Asn Ile Asn
1               5                   10                  15

Glu Tyr Ser Lys Glu Glu Asn Lys Leu
            20                  25

<210>  112
<211>  33
<212>  PRT
<213>  Plasmodium falciparum

<400>  112

Thr Ser Phe Ser Lys Tyr Val Arg Gln Leu Glu Gln Tyr Phe Asp Asn
1               5                   10                  15

Phe Asp Gln Asp Phe Leu Ser Leu Arg Gln Lys Ile Ser Asp Ile Leu
            20                  25                  30

Gln

<210>  113
<211>  30
<212>  PRT
<213>  Plasmodium falciparum

<400>  113

Glu Lys Leu Val Lys His Leu Asp Val Ile Asp Lys Leu Ile Glu Asn
1               5                   10                  15

Ile Tyr Asp Asn Ile Asn Asn Leu Asn Glu Tyr Ile Asn Lys
            20                  25                  30

<210>  114
<211>  25
<212>  PRT
<213>  Plasmodium falciparum

<400>  114

Ile Asn Ser His Asn Asn Asn Met Asn Asn Asn Asn Asp Asn Met Asn
1               5                   10                  15

Asn Met Asn Asn Asn Asn Asn Asn Ile
            20                  25

<210>  115
<211>  25
<212>  PRT
<213>  Plasmodium falciparum

<400>  115

Ile Asn Glu Ile Glu Lys Lys Ile Glu Asp Ile Glu Lys Asn Ile Asn
1               5                   10                  15

Ile Thr Lys Glu Asn Leu Lys Glu Leu
            20                  25

<210>  116
<211>  25
<212>  PRT
<213>  Plasmodium falciparum

<400>  116

Leu Lys Glu Leu Glu Asn Lys Ile Thr Glu Leu Gln Ser Ser Phe Ser
1               5                   10                  15

Ser Tyr Glu Asn Glu Met Lys His Val
            20                  25

<210>  117
<211>  25
<212>  PRT
<213>  Plasmodium falciparum

<400>  117

Ile Asp Asp Ile Asp Arg Asn Ala Glu Gln Ile Asn Lys Asn Tyr Glu
1               5                   10                  15

His Val Asn Lys Asn Tyr Glu His Val
            20                  25

<210>  118
<211>  25
<212>  PRT
<213>  Plasmodium falciparum

<400>  118

Ile Asp Arg Gln Arg Asp Lys Ile Asn Glu Leu Glu Lys Lys Leu Glu

1               5                   10                  15


Glu Leu Arg Ser Ser Ser Glu Glu Leu
            20                  25


<210>  119
<211>  37
<212>  PRT
<213>  Plasmodium falciparum

<400>  119

Thr Ile Val Gln Asn Ser Tyr Asn Ser Phe Ser Asp Ile Asn Lys Asn
1               5                   10                  15


Ile Asn Asp Ile Asp Lys Glu Met Lys Thr Leu Ile Pro Met Leu Asp
            20                  25                  30


Glu Leu Leu Asn Glu
        35


<210>  120
<211>  25
<212>  PRT
<213>  Plasmodium falciparum

<400>  120

Ile Ser Glu Leu Glu Gln Glu Phe Asn Asn Asn Asn Gln Lys Leu Asp
1               5                   10                  15


Asn Ile Leu Gln Asp Ile Asn Ala Met
            20                  25


<210>  121
<211>  32
<212>  PRT
<213>  Plasmodium falciparum

<400>  121

Met Glu Ile Lys Thr Ile Val Gln Asn Ser Tyr Asn Ser Phe Ser Asp
1               5                   10                  15


Ile Asn Lys Asn Ile Asn Asp Ile Asp Lys Glu Met Lys Thr Leu Ile
            20                  25                  30


<210>  122
<211>  25
<212>  PRT
<213>  Plasmodium falciparum

<400>  122

Met Leu Ser Leu Gln Asn Asn Ile Asp Lys Leu Lys Lys Ser Asn Asn
1               5                   10                  15

Asn Leu Asn Glu Asp Leu Arg Lys Lys
            20                  25

<210>  123
<211>  25
<212>  PRT
<213>  Plasmodium falciparum

<400>  123

Met Asn Leu Leu Arg Glu Ile Leu Lys Leu Met Thr Asp Asn Ile Asp
1               5                   10                  15

Thr Leu Lys Asp Lys Ile Asn Glu Ile
            20                  25

<210>  124
<211>  29
<212>  PRT
<213>  Plasmodium falciparum

<400>  124

Gly Gly Leu Lys Asn Ser Asn His Asn Leu Asn Asn Ile Glu Met Lys
1               5                   10                  15

Tyr Asn Thr Leu Asn Asn Asn Met Asn Ser Ile Asn Lys
            20                  25

<210>  125
<211>  33
<212>  PRT
<213>  Plasmodium falciparum

<400>  125

Pro Tyr Leu Arg Arg Ala Lys His Asn Leu Asn Asn Leu Gln Gly Gly
1               5                   10                  15

Ile Asn Asn Leu Tyr Ser Ser Val Asn Val Val Tyr Asp Asn Leu Phe
            20                  25                  30

Asn

<210>  126
<211>  25
<212>  PRT
<213>  Plasmodium falciparum

<400>  126

Leu Asn Ser Leu Glu Arg Thr Val Ala Ser Leu Lys Asn Asn Glu Lys
1               5                   10                  15

Gln Leu His Asn Asn Ile Gln Lys Ile
            20                  25

<210>  127
<211>  32
<212>  PRT
<213>  Plasmodium falciparum

<400>  127

Ser Leu Leu Asp Thr Leu Glu Lys Ser Val Lys Gly Ile Asp Glu Asn
1               5                   10                  15

Ile Glu Lys Tyr Asn Lys Glu Leu Asn Val Ile Lys Gln Lys Ile Glu
            20                  25                  30

<210>  128
<211>  31
<212>  PRT
<213>  Plasmodium falciparum

<400>  128

Asn Asn Glu Met Asp Glu Thr Ile Asn Lys Leu Lys Lys Asp Ile Asn
1               5                   10                  15

Lys Leu Asn Glu Lys Ile Glu Lys Tyr Asp Asn Phe Met Lys Met
            20                  25                  30

<210>  129
<211>  25
<212>  PRT
<213>  Plasmodium falciparum

<400>  129

Ile Asp Glu Leu Glu Asn Lys Ile Glu Lys Leu Lys Asn Glu Leu Ser
1               5                   10                  15

Lys Asn Ser His Asn Asn Asn Asn Ile
            20                  25

<210>  130
<211>  25
<212>  PRT
<213>  Plasmodium falciparum

<400>  130

Ile Asn Asn Ile Asp Asp His Ile Asn Asn Ile Asp Asp His Ile Asn
1               5                   10                  15

Asn Ile Asp Asp His Ile Asn Asn Ile
            20                  25

<210>  131
<211>  25
<212>  PRT
<213>  Plasmodium falciparum

<400>  131

Ile Asn Asn Ile Asp Asp Asn Lys Asn Asn Ile Asp Asp His Ile Asn
1               5                   10                  15

Asn Ile Asp Asp His Ile Asn Asn Ile
            20                  25

<210>  132
<211>  25
<212>  PRT
<213>  Plasmodium falciparum

<400>  132

Leu Lys Ser Leu Asn Glu Lys Ile Lys Asn Tyr Asp Ser Ile Ile Glu
1               5                   10                  15

Glu Gln Lys Asn Gln Leu Glu Asn Leu
            20                  25

<210>  133
<211>  140
<212>  PRT
<213>  Plasmodium falciparum

<400>  133

Met Ile Lys Asn Pro Tyr Ser Lys Ile Lys Asn Glu Lys Ser Phe Ile
1               5                   10                  15

Asp Leu Asn Phe Ile Lys Gly Tyr Ser Asn Phe Ile Lys Lys Lys Cys
            20                  25                  30

Val Pro Tyr Asp Lys Lys Lys Tyr Ile Phe Cys Asn Lys Asn Arg Leu
        35                  40                  45

Ile Ser Asn Val Gly Asn Lys Ile Asn Lys Lys Glu Ser Glu Glu Gln
    50                  55                  60

Tyr Asp Ser Asp Asp Asp Asn Asp Lys Leu Val Leu Asn Asp Asp Glu
65                  70                  75                  80

Asp Asp Glu Lys Lys Gln Val His Ile Asn Asn Lys Thr Glu Ala Thr
                85                  90                  95

Asn Ile Thr Asn Ile Asn Lys Asn Ile Glu Asn Ile Lys Asn Asp Met
            100                 105                 110

Ser Asn Leu Asn Asn Met Asn Asp Ser Asn Gln Lys Ile Lys Met Lys
        115                 120                 125

Asn Lys Gly Lys Tyr Asp Ser Asp Val Ile Ile Met
    130                 135                 140

## Description

### FIELD OF THE INVENTION

This invention relates generally to the field of pathogen peptidic antigens and their use, for example, for the preparation of a vaccine against said pathogen. More specifically, the present invention relates to an antigenic peptide deriving from plasmodium species sequences, and includes antibodies and methods of producing and using same.

### BACKGROUND OF THE INVENTION

Human Plasmodium falciparum (*Pf*) is a public health problem. Today approximately 40% of the world's population mostly those living in the poorest countries are at risk of malaria. Malaria causes more than 300 million acute illnesses and at least one million deaths annually. 90% of deaths due to malaria occur in sub-Saharan African countries mostly among young children and pregnant women (http://rbm.who.int).

Unfortunately, vaccine discovery is still a very empirical process given that protective antigens do not bear any structural, physicochemical or sequence related characteristics that would allow their identification. The only constraint for antigens whose protective capacity is antibody mediated is their accessibility to antibody molecules. Initially, vaccine development was performed with intact microorganisms or proteins, which were easily obtainable through classical purification methods. Introduction of molecular biology techniques generally improved the antigen isolation step, but difficulty in producing highly purified recombinant proteins in milligram amounts still remains; this renders this approach very costly and not adaptable to high throughput screening methods. Furthermore, proper protein folding still represents an additional problem in numerous cases. Thus, overcoming the bottlenecks of manufacturing and identification of proteins as targets of protection represents a grand challenge to the scientific community.

In the past 20 years huge efforts have been made to develop a malaria vaccine with over 40 phase I and II trials being conducted (Ballou *et al,* 2004, Van de Perre and Dedet, 2004). Currently, there are approximately 70 different vaccine candidates under development with at least 10 in clinical trials. The antigens used are derived from various stages of the parasite life cycle (Genton and Corradin, 2002, Ballouet al, 2004), with various immunisation regimens (Dunachie and Hill, 2003) and combined with different adjuvants formulated in various delivery systems (Genton *et al,* 2002; Taylor-Robinson, 2003; Ballou *et al,* 2004).

Results obtained in clinical trials conducted in endemic situations were encouraging and showed some levels of protection (Bojang *et al,* 1998, Bojang *et al,* 2001, Gentonet *et al,* 2002) while other trials showed no protection (Nosten *et al,* 1996, Valero *et al,* 1996, Urdaneta *et al,* 1998, Graves and Gelband, 2004). To date, the most advanced malaria vaccine is RTS,S/AS02A. This vaccine comprises a large portion of the immunologically dominant NANP repeats of the circumsporozoite protein and a portion of the C-terminal region containing T cell epitopes, which is fused to hepatitis B surface antigen (Gordon *et al,* 1995), co-expressed in *Saccharomyces cerevisiae* and formulated with the strong adjuvant AS02A (Stoute *et al,* 1997). RTS,S/AS02A targets the pre-erythrocytic liver stage and protects against both clinical malaria and parasitaemia in naive (Stoute *et al,* 1997) and malaria exposed adult males (Bojang *et al,* 2001), although for a short period. In addition, better protection level against clinical malaria has been obtained in toddlers in a recently completed trial in Mozambique (Alonso *et al,* 2004). Protection was sustained for up to 6 months in children, which are the most affected by the disease.

The vaccine candidates that have been used to date have not been systematically selected for their ability to protect; most of the parasite molecules were empirically prioritised following their chronological order of discovery. This approach though successful for other pathogens, has not been able to provide an effective malaria vaccine.

For example, EP 0315 0185 B1 (in the name of Behringwerke aktiengesellschaft) described the isolation of a gene which codes for a histidine/alanine-rich protein by screening two different Plasmodium falciparum cDNA gene banks with antiserum against a 41kDa protein and by cross-hybridisation with the insert DNA of a clone obtained therewith. The isolated protein protected Aotus monkeys from infection with Plasmodium falciparum. Unfortunately, this approach had not lead to the development of an efficient vaccine in humans probably due to the fact that these monkeys are not the appropriate models to mimic human immunological response to Plasmodium falciparum.

In an attempt to develop vaccines against another pathogen, Streptococcus pneumoniae, mapping studies using monoclonal antibodies raised against pneumococcal surface protein A (PspA) indicated that the major cross-reactive epitopes are found in the last 100 amino-acids of the coiled-coil domain which is an important structural and biologically abundant domain found in diverse group of proteins (Chen and Parry, 1986 and 1990).

Pursuant to this finding, International Patent Application WO00/37105 (in the name of Medimmune, Inc.) disclosed a process and a vaccine composition for preventing infection caused by Streptococcus pneumoniae that comprises polypeptides and fragments of polypeptides containing histidine triad residues or coiled-coil regions.

More recently, International Patent Application WO01/96368 (in the name of Cytovax Biotechnologies, Inc.) disclosed the use of a coiled-coil structural scaffold to generate structure-specific peptides, including synthetic peptides. These synthetic peptides are useful as vaccines or to stimulate antibody production or cell-mediated immunity to the naturally occurring pneumococcal surfaces proteins A and C.

Although there has been some progress in the treatment of malaria, the development of a safe and effective malaria vaccine remains an urgent unmet medical need for vast populations living in malaria-endemic region.

This object has been achieved by providing a new antigenic peptide deriving from Plasmodium species comprising at least one coiled coil region. This peptide has been prepared according to a method of producing an antigenic peptide for the preparation of a vaccine against a pathogen based on a new approach for identifying the presence of a sequence encoding at least one coiled-coil region in the genome of said pathogen.

### SUMMARY OF THE INVENTION

These and other objects as will be apparent from the foregoing have been achieved by providing a new antigenic peptide deriving from Plasmodium species comprising at least one coiled coil region, characterized in that said antigenic peptide is selected from the group comprising the amino acid sequences SEQ ID N°1 to SEQ ID N°131, fragments thereof, molecular chimeras thereof, combinations thereof and/or variants thereof.

Another object of the invention is to provide an antigenic cocktail composition deriving from Plasmodium species.

This invention also contemplates the use of the antigenic peptide deriving from Plasmodium species or of the antigenic cocktail composition in the preparation of a vaccine composition useful to stimulate an immune response in a mammal and the use thereof in the manufacture of a medicament for the treatment and/or prevention of malaria

A further object of the present invention is to provide an antibody that recognizes the antigenic peptide of or the antigenic cocktail composition deriving from Plasmodium species.

The present invention also relates to a purified and isolated nucleic acid sequence comprising a nucleotidic sequence encoding the antigenic peptide deriving from Plasmodium species, an expression vector comprising at least one copy of said purified and isolated nucleic acid sequence, and a host cell comprising either the purified and isolated nucleic acid sequence or the expression vector.

The present invention further relates to a method of producing an antigenic peptide for the preparation of a vaccine composition against a pathogen.

A diagnostic tool for determining the presence of the antigenic peptide or antibodies directed against said antigenic peptide is also contemplated in the present invention

Other objects and advantages will become apparent to those skilled in the art from a review of the ensuing detailed description, which proceeds with reference to the following illustrative drawings, and the attendant claims.

### BRIEF DESCRIPTION OF THE FIGURES

**Figure 1** represents the immunofluorence data obtained on infected human erythrocytes using affinity purified human antibodies.
**Figure 2** represents the immunofluorence data obtained on malaria infected human erythrocytes using specific mouse antibodies or monoclonal mouse antibodies.

### DETAILED DESCRIPTION OF THE INVENTION

The present invention relates to an antigenic peptide deriving from Plasmodium species comprising at least one coiled coil region from a Plasmodium species sequence.

The term "comprising" is generally used in the sense of including, that is to say permitting the presence of one or more features or components.

As used herein, the terms "protein", "polypeptide", "polypeptidic", "peptide" and "peptidic" are used interchangeably herein to designate a series of amino acid residues connected to the other by peptide bonds between the alpha-amino and carboxy groups of adjacent residues.

The term "antigenic peptide" refers to a peptide that is recognized by an antibody. Usually, the immune response results in the production of antibodies recognizing the antigenic peptide or at least a part thereof.

In the present context, the term "deriving" refers to the fact that the antigenic peptide has been selected among the amino acid sequences present in the Plasmodium species pathogen sequence.

"Coiled coil region", "coiled-coil domain" or "region" are usually made up of amino acid sequence having heptad (*abcdefg*) repeats with apolar residues at the a and b positions and typically polar residues elsewhere. The structure of these regions are stabilized by hydrophobic interactions between the *a* and *d* positions in the different strands (Hodges et al, 1981; Hodges, 1996).

"Plasmodium species" as used herein can be selected among the following plasmodium strains:
Plasmodium agamae, Plasmodium atheruri, Plasmodium azurophilum, Plasmodium berghei, Plasmodium brasilianum, Plasmodium cathemerium, Plasmodium chabaudi, Plasmodium chiricahuae, Plasmodium coatneyi, Plasmodium cuculus, Plasmodium cynomolgi, Plasmodium elongatum, Plasmodium fairchildi, Plasmodium falciparum, (such as Plasmodium falciparum (isolate 311), Plasmodium falciparum (isolate 7G8) ,Plasmodium falciparum (isolate CAMP / Malaysia), Plasmodium falciparum (isolate CDC / Honduras), Plasmodium falciparum (isolate DD2), Plasmodium falciparum (isolate FC27 / Papua New Guinea), Plasmodium falciparum (isolate FcB1 / Columbia), Plasmodium falciparum (isolate FCBR / Columbia), Plasmodium falciparum (isolate FCH-5), Plasmodium falciparum (isolate FCM17 / Senegal), Plasmodium falciparum (isolate FCR-3 / Gambia), Plasmodium falciparum (isolate fid3 / india), Plasmodium falciparum (isolate hb3), Plasmodium falciparum (isolate IMR143), Plasmodium falciparum (isolate K1 / Thailand), Plasmodium falciparum (isolate KF1916), Plasmodium falciparum (isolate LE5), Plasmodium falciparum (isolate mad20 / papua new guinea), Plasmodium falciparum (isolate mad71 / papua new guinea), Plasmodium falciparum (isolate NF54), Plasmodium falciparum (isolate NF7 / Ghana), Plasmodium falciparum (isolate nig32 / nigeria), Plasmodium falciparum (isolate PALO ALTO / UGANDA), Plasmodium falciparum (isolate RO-33 / Ghana), Plasmodium falciparum (isolate T4 / Thailand), Plasmodium falciparum (isolate TAK 9), Plasmodium falciparum (isolate thtn / thailand), Plasmodium falciparum (isolate V1), Plasmodium falciparum (isolate WELLCOME), Plasmodium falciparum 3D7), Plasmodium fieldi, Plasmodium floridense, Plasmodium fragile ,Plasmodium gallinaceum, Plasmodium giganteum, Plasmodium gonderi, Plasmodium guanggong, Plasmodium heteronucleare, Plasmodium hylobati, Plasmodium inui, Plasmodium juxtanucleare, Plasmodium knowlesi ,Plasmodium lophurae, Plasmodium malariae, Plasmodium cf. malariae, Plasmodium mexicanum, Plasmodium nucleophilum, Plasmodium ovale, (malaria parasite P. ovale), Plasmodium reichenowi, Plasmodium relictum, Plasmodium rouxi, Plasmodium simiovale, Plasmodium simium, Plasmodium vinckei, Plasmodium vivax (such as Plasmodium vivax (strain Belem) Plasmodium vivax (strain Salvador I)), Plasmodium yoelii, Plasmodium sp. unclassified such as Plasmodium sp. 909413, Plasmodium sp. 909414, Plasmodium sp. 909415, Plasmodium sp. 909416, Plasmodium sp. AP61, Plasmodium sp. AP62, Plasmodium sp. AP63, Plasmodium sp. AP64, Plasmodium sp. AP65, Plasmodium sp. AP66, Plasmodium sp. AP67, Plasmodium sp. AP68, Plasmodium sp. AP69, Plasmodium sp. AP70, Plasmodium sp. AP71, Plasmodium sp. AP72, Plasmodium sp. AP73, Plasmodium sp. AP74, Plasmodium sp. AP75, Plasmodium sp. AP76, Plasmodium sp. AP77, Plasmodium sp. AP78, Plasmodium sp. C1, Plasmodium sp. C2, Plasmodium sp. C3, Plasmodium sp. COLL1, Plasmodium sp. D1, Plasmodium sp. DAJ-2004, Plasmodium sp. E1, Plasmodium sp. E2, Plasmodium sp. ex Aplonis atrifusca, Plasmodium sp. ex Aplonis tabuensis, Plasmodium sp. ex Foulehaio carunculata, Plasmodium sp. ex Halcyon chloris, Plasmodium sp. ex Myzomela cardinalis, Plasmodium sp. ex Ptilinopus porphyraceus, Plasmodium sp. F1, Plasmodium sp. G1, Plasmodium sp. GRW11, Plasmodium sp. GRW2, Plasmodium sp. GRW4, Plasmodium sp. H1, Plasmodium sp. ORW1, Plasmodium sp. PA, Plasmodium sp. PARUS2, Plasmodium sp. PB, Plasmodium sp. pBT6, Plasmodium sp. pBT7, Plasmodium sp. pBT8, Plasmodium sp. pBT9, Plasmodium sp. PC, Plasmodium sp. pGRW9, Plasmodium sp. PH ,Plasmodium sp. PI, Plasmodium sp. PU1, Plasmodium sp. SGS1 , Plasmodium sp. strain KZ02, Plasmodium sp. strain LA09, Plasmodium sp. strain LA15, Plasmodium sp. strain OZ01A, Plasmodium sp. strain OZ04, Plasmodium sp. strain OZ08, Plasmodium sp. strain OZ14, Plasmodium sp. strain OZ25, Plasmodium sp. strain OZ35, Plasmodium sp. strain OZ35A, Plasmodium sp. strain OZ35B, Plasmodium sp. strain OZ36A, Plasmodium sp. strain OZ42, Plasmodium sp. strain OZ45, Plasmodium sp. strain OZ46, Plasmodium sp. strain PR01, Plasmodium sp. SYAT24, Plasmodium vivax-like sp).

Preferably, the antigenic peptide will derive from Plasmodium falciparum, Plasmodium vivax, Plasmodium ovale and Plasmodium malariae.

Usually, the antigenic peptide deriving from Plasmodium species comprising at least one coiled coil region will be selected from the group comprising the amino acid sequences SEQ ID N°1 to SEQ ID N° 131, fragments thereof, molecular chimeras thereof, combinations thereof and/or variants thereof.

Applicants have selected SEQ ID N°1 to SEQ ID N°131, represented in table 1, according to the method of producing an antigenic peptide described *infra.* All these 131 peptidic sequences contain at least one coiled coil region.

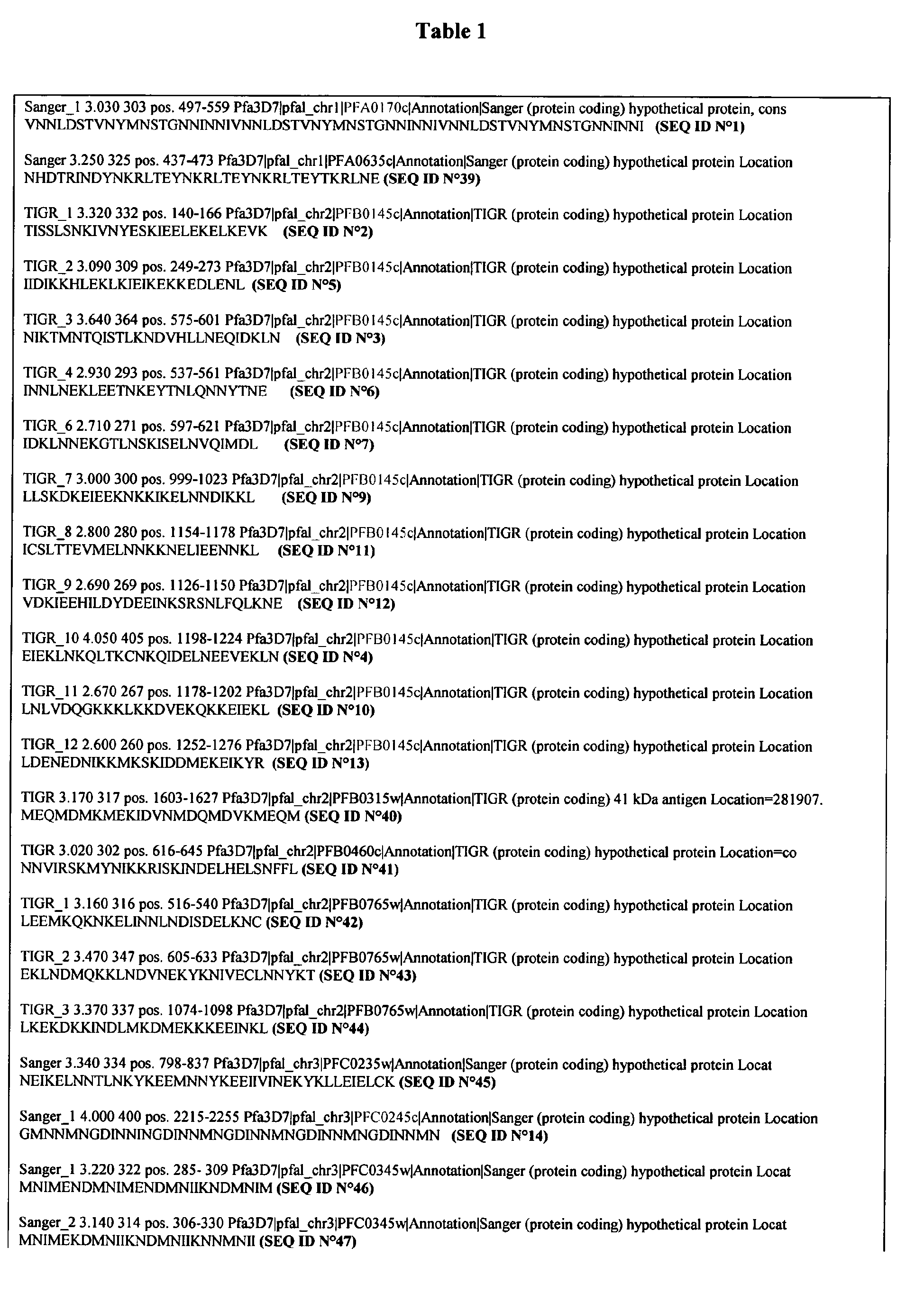

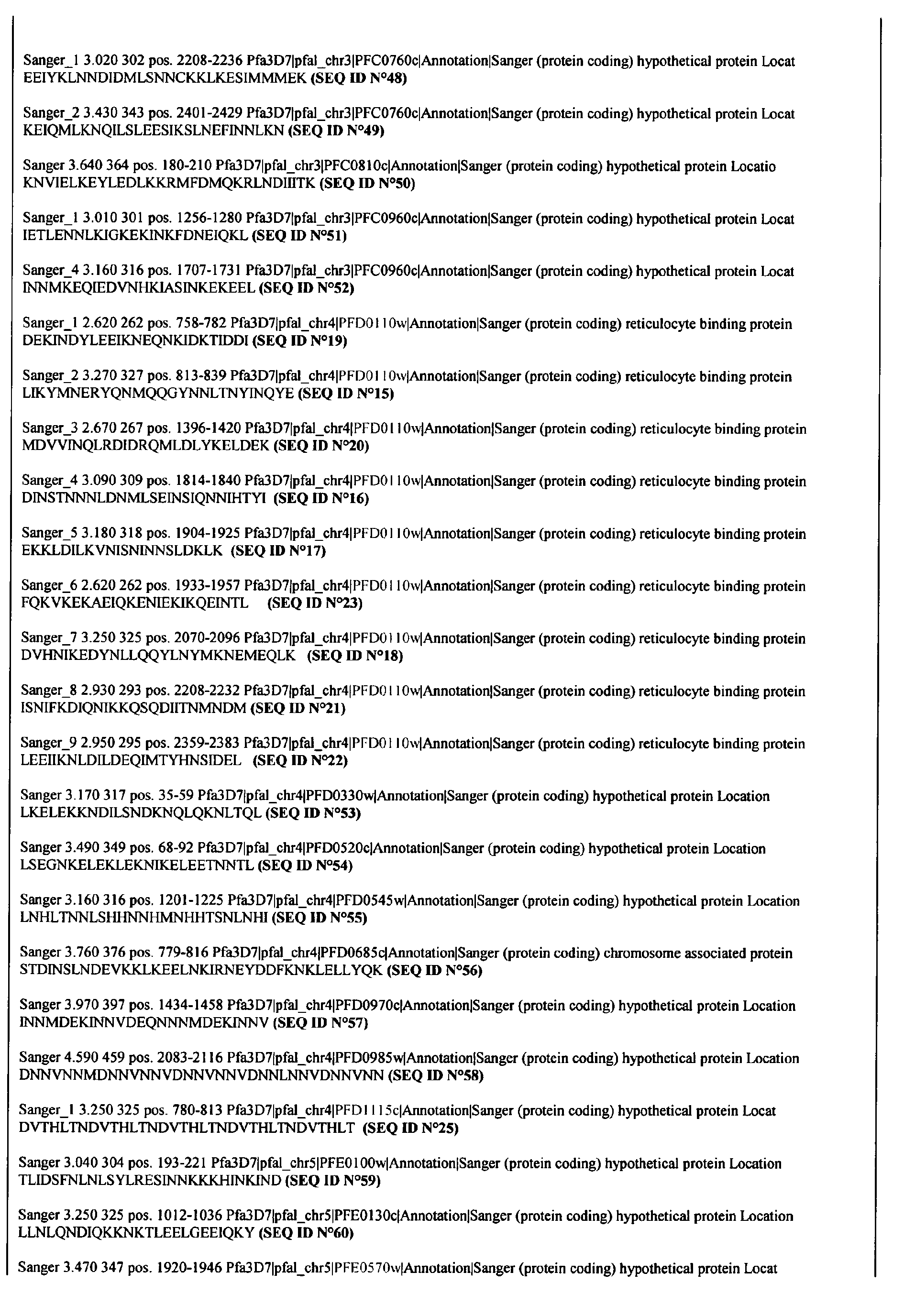

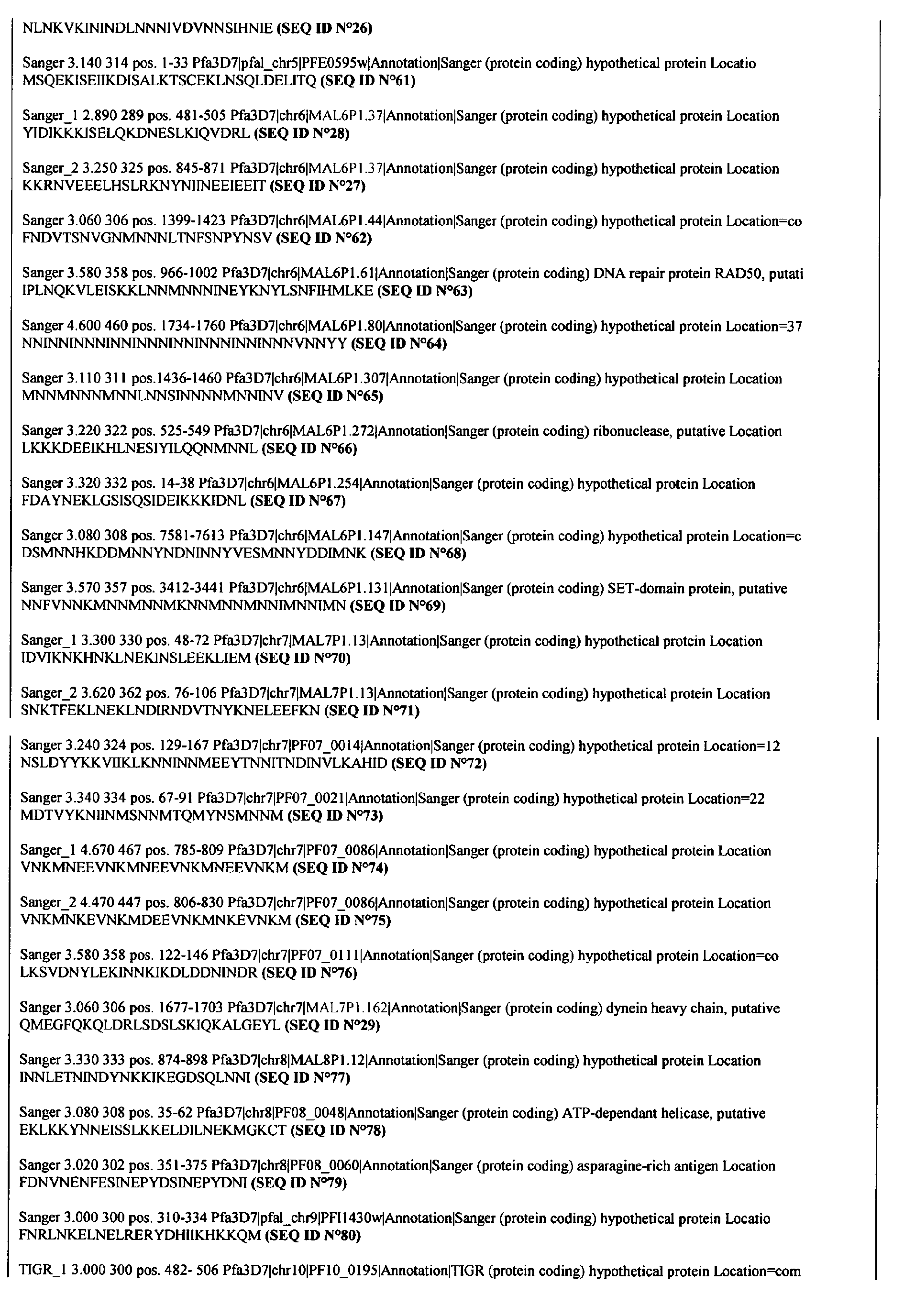

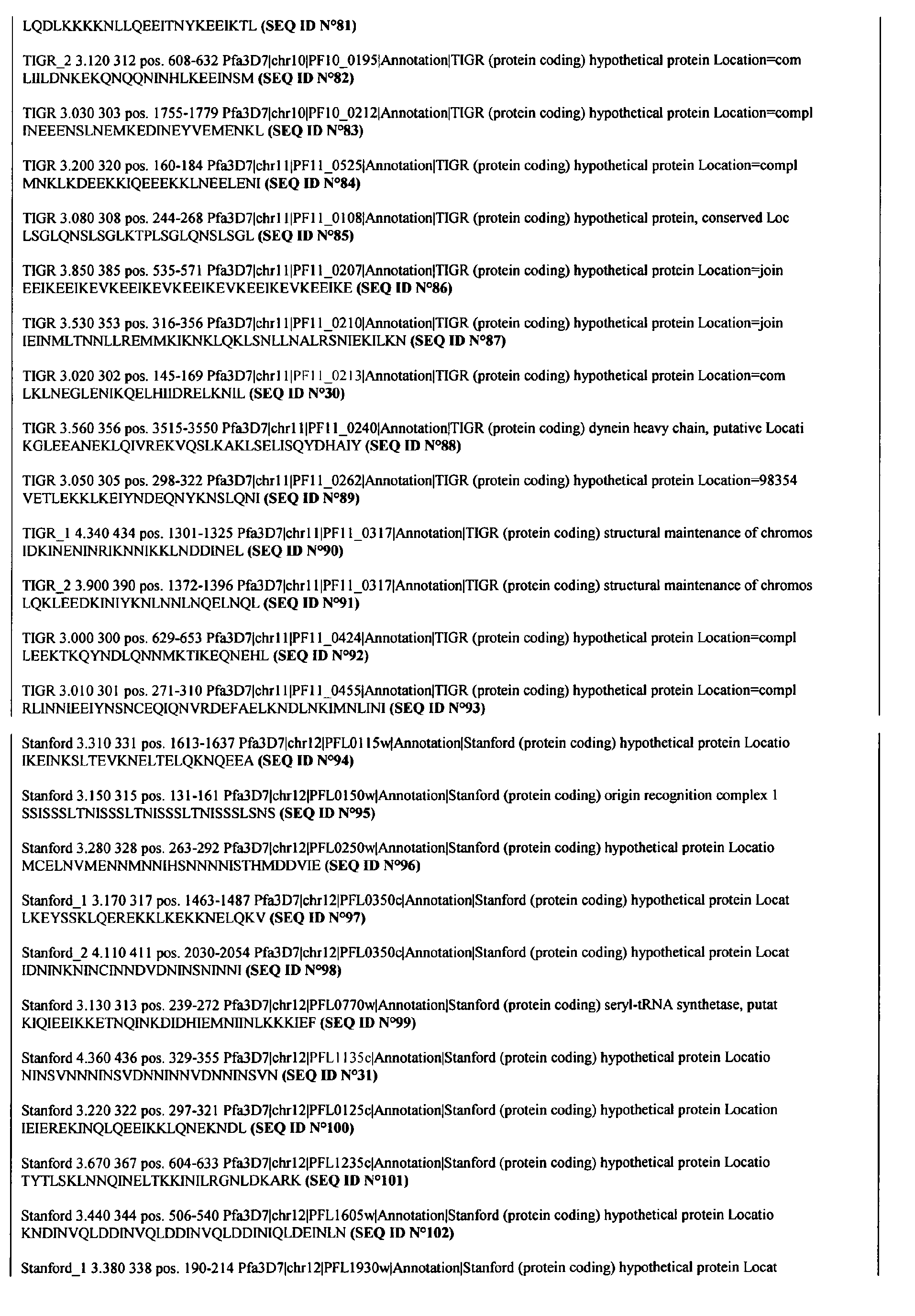

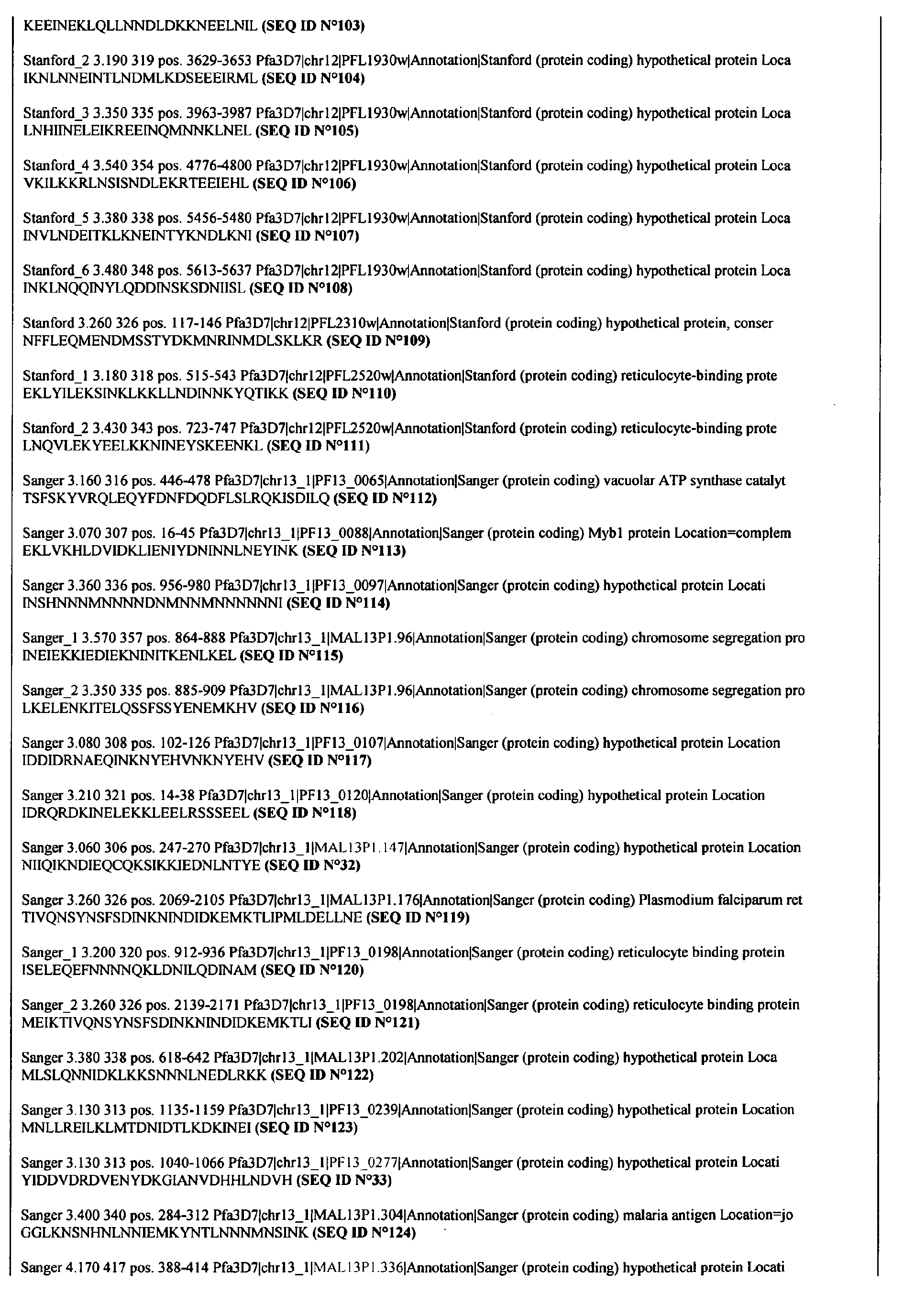

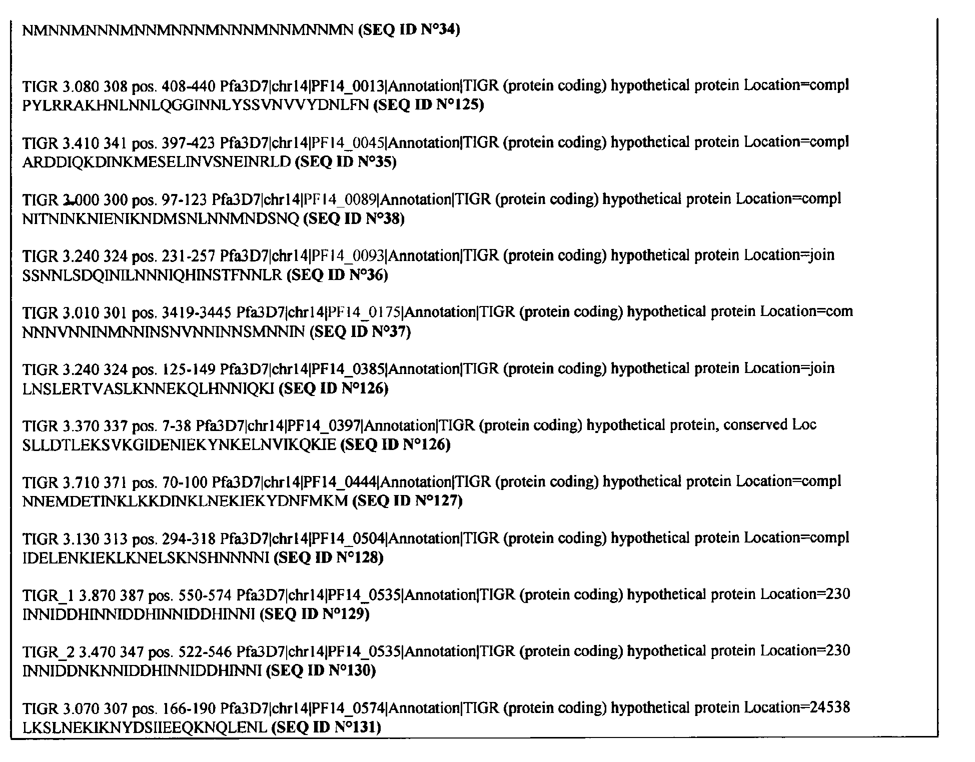

Among these 131 sequences, 38 coiled-coil segments were randomly selected (SEQ ID N°1 to 38, 30-40 amino acids long with the highest coiled-coil score) and are present either in the same protein or in different ones. All these 38 selected antigenic peptides were recognized at various degrees (5-60%) by a panel of sera from donors living in endemic areas (Table 2).

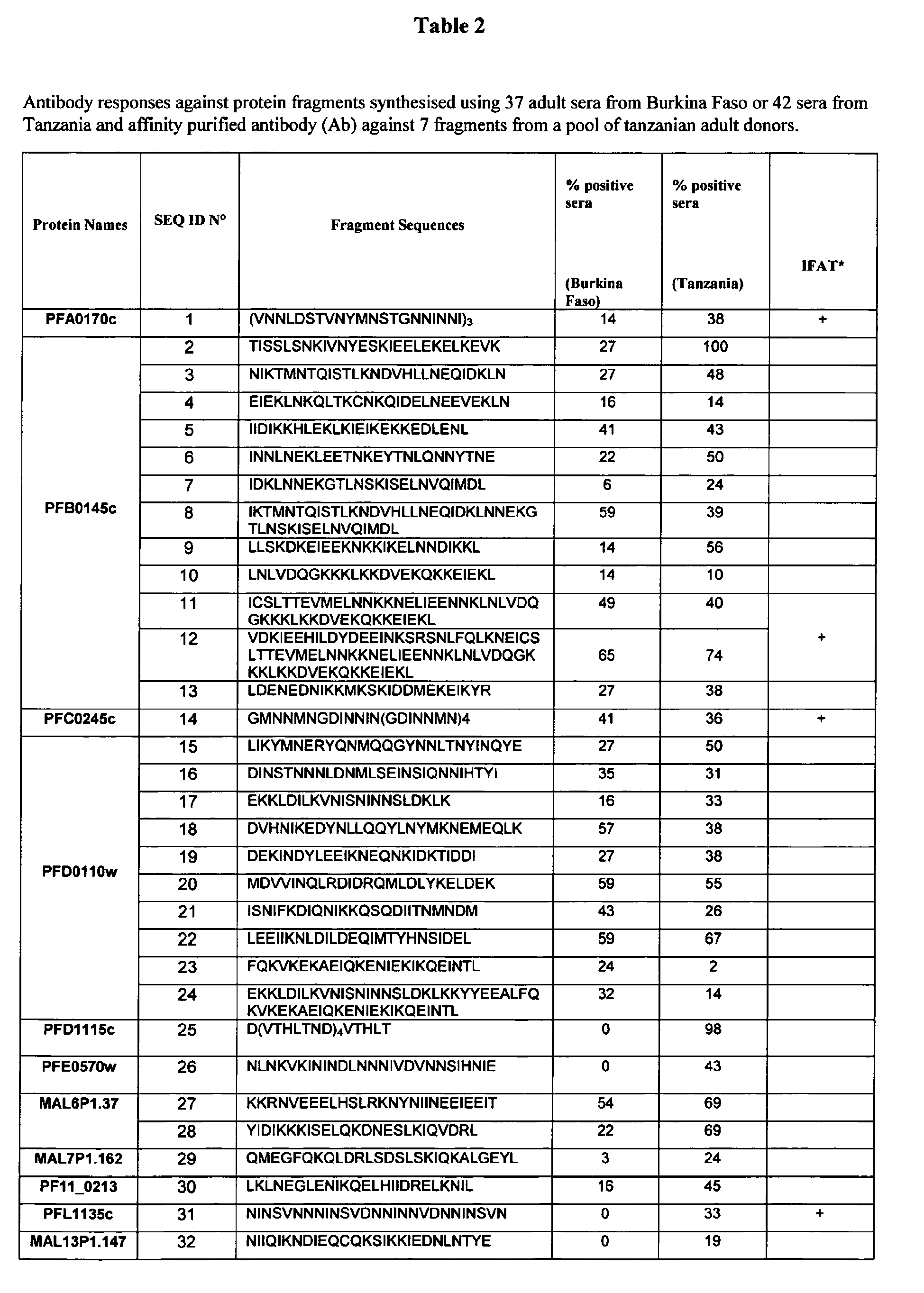

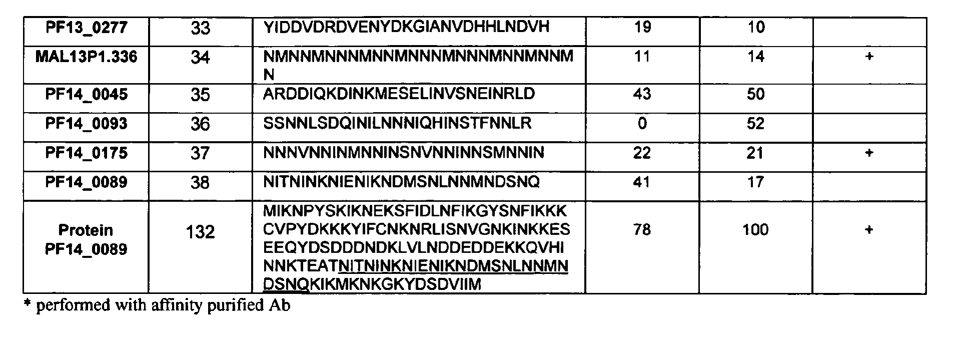

Thus, 17 new proteins were identified with length varying from 200 to 10,000 amino acids. One of these proteins (protein PF14_0089) containing antigenic peptide (SEQ ID N°38) was chemically synthesized and tested for recognition in ELISA assays (see Table 2). This novel protein is recognized by 78-100% of adult sera from Burkina Faso and Tanzania. Affinity purified antibodies recognize parasite infected red blood cells. In addition, this protein has also been associated with protection in Tanzanian children (data not shown).

"Fragments" refer to a part of a sequence containing less amino acids in length than the sequence of the peptide of the invention. This sequence can be used as long as it exhibits the same properties as the native sequence from which it derives. Preferably this sequence contains less than 30%, preferably less than 60%, in particular less than 90% amino acids in length than the respective sequence of the peptide of the invention. These sequences can be used as long as they exhibit the same properties as the native sequence from which they derive.

These fragments can be prepared by a variety of methods and techniques known in the art such as for example chemical synthesis (e.g. multi-channel peptide synthesizer).

Furthermore, since an inherent problem with native peptides (in L-form) is the degradation by natural proteases, the peptide of the invention may be prepared in order to include D-forms and/or "retro-inverso isomers" of the peptide. Preferably, retro-inverso isomers of short parts, variants or combinations of the peptide of the invention are prepared.

By "retro-inverso isomer" is meant an isomer of a linear peptide in which the direction of the sequence is reversed and the chirality of each amino acid residue is inverted; thus, there can be no end-group complementarity.

Protecting the peptide from natural proteolysis should therefore increase the effectiveness of the specific heterobivalent or heteromultivalent compound. A higher biological activity is predicted for the retro-inverso containing peptide when compared to the non-retro-inverso containing analog owing to protection from degradation by native proteinases. Furthermore they have been shown to exhibit an increased stability and lower immunogenicity [Sela M. and Zisman E., (1997) Different roles of D-amino acids in immune phenomena- FASEB J. 11, 449].

Retro-inverso peptides are prepared for peptides of known sequence as described for example in Sela and Zisman, (1997).

Also encompassed by the present invention are modifications of the antigenic peptide (which do not normally alter primary sequence), including in vivo or in vitro chemical derivitization of peptides, e.g., acetylation or carboxylation. Also included are modifications of glycosylation, *e.g.*, those made by modifying the glycosylation patterns of a peptide during its synthesis and processing or in further processing steps, e. g., by exposing the peptide to enzymes which affect glycosylation e. g. , mammalian glycosylating or deglycosylating enzymes. Also included are sequences which have phosphorylated amino acid residues, e. g., phosphotyrosine, phosphoserine, or phosphothreonine.

Encompassed by the present invention is also a molecular chimera of the antigenic peptide of the invention. By "molecular chimera" is intended a polynucleotide sequence that may include a functional portion of the antigenic peptide and that will be obtained, for example, by protein chemistry techniques known by those skilled in the art.

Particular combinations of the antigenic peptide sequence or fragments or subportions thereof are also considered in the present invention.

The present invention also includes variants of the antigenic peptide. The term "variants" refer to polypeptides having amino acid sequences that differ to some extent from a native sequence polypeptide, that is amino acid sequences that vary from the native 3D sequence whereby one or more amino acids are substituted by another one. The variants can occur naturally (e.g. polymorphism) or can be synthesized. Variants possess substitutions, deletions, and/or insertions at certain positions within the amino acid sequence of the native amino acid sequence. Amino acid substitutions are herein defined as exchanges within one of the following five groups:
I. Small aliphatic, nonpolar or slightly polar residues: Ala, Ser, Thr, Pro, Gly
II. Polar, positively charged residues: His, Arg, Lys
III. Polar, negatively charged residues: and their amides: Asp, Asn, Glu, Gln
IV. Large, aromatic residues: Phe, Tyr, Trp
V. Large, aliphatic, nonpolar residues: Met, Leu, Ile, Val, Cys.

Non-natural amino acids can also be introduced by chemical synthesis.

The present invention also relates to an antigenic cocktail composition of Plasmodium species that comprises at least 2 antigenic peptides of the invention or mixtures thereof. Applicants have shown that the use of an antigenic cocktail composition of the invention improves the performance of vaccines since simultaneous immune responses to antigens, which are involved in the same or in different pathogenic mechanisms, surprisingly confers greater and sustained protection. The antigenic peptides may be free or linked together. In case said antigenic peptides are linked, they are preferably linked together by the way of a linker such as PEG (Poly ethylene glycol).

Also encompassed by the present invention is the use of an antigenic peptide or of the antigenic cocktail composition of the invention for the preparation of a vaccine composition useful to stimulate an immune response in a mammal. "Mammal" refers to any animal classified as a mammal including humans, domestic and farm animals, and zoo, sports or pet animals, such as dogs, horses, cats, cows, monkeys, etc. Preferably the mammal is a human.

To augment the immune response elicited, it may be preferable to couple the peptides of SEQ ID NOS: 1-131, or the antigenic cocktail composition, to any carrier molecule or carrier proteins. Various protein, glycoprotein, carbohydrate or sub-unit carriers can be used, including but not limited to, tetanus toxoid/toxin, diphtheria toxoid/toxin, pseudomonas mutant carrier, bacteria outer membrane proteins, crystalline bacterial cell surface layers, various endo or exotoxins, serum albumin, gamma globulin or keyhole limpet hemocyanin, recombinant, exotoxin A, LT toxin, Cholera B toxin, Klebsiella pneumoniae OmpA, Bacterial flagella, Clostridium difficile recombinant toxin A, Peptide dendrimers (multiple antigenic peptides), pan DR epitope (PADRE), universal T-cell epitopes from tetanus toxin, Commensal bacteria, Phage (displaying peptide on and bacteria phages), attachment of peptides to recombinant IgGl and/or other suitable constituents.

In addition, the peptides of SEQ ID NOS: 1-131, or the antigenic cocktail composition, or their conjugates with carrier proteins may be further mixed with adjuvants to elicit an immune response, as adjuvants may increase immunoprotective antibody titers or cell mediated immunity response. Such adjuvants can include, but are not limited to, MPL + TDM + CWS (SIGMA), MF59 (an oil-in-water emulsion that includes 5% squalene, 0.5% sorbitan monoleate and 0.5% sorbitan trioleate Chiron), Heat-labile toxin (HLT), CRMig (nontoxic genetic mutant of diphtheria toxin), Squalene (IDEC PHARMACEUTICALS CORP.), Ovalbumin (SIGMA), Quil A (SARGEANT, INC.), Aluminum phosphate gel (SUPERFOS BIOSECTOR), Cholera holotoxin (CT LIST BIOLOGICAL LAB.), Cholera toxin B subunit (CTB), Cholera toxin A subunit- Protein A D-fragment fusion protein, Muramyl dipeptide (MDP), Adjumera (polyphosphazene, VIRUS RESEARCH INSTITUTE), SPT (an emulsion of 5% squalene, 0.2% Tween 80, 1. 25% Pluronic L121 with phosphate-buffered saline ph 7. 4), Avridine (M6 PHARMACEUTICALS), Bay R1005 (BAYER), Calcitrol (SIGMA), Calcium phosphate gel (SARGEANT INC.), CRL 1005 (Block co-polymer P1205, VAXCEL CORP.), DHEA (MERCK), DMPC (GENZYME PHARMACEUTICALS and FINE CHEMICALS). DMPG (GENZYME PHARMACEUTICALS and FINE CHEMICALS), Gamma Inulin, Gerbu Adjuvant (CC BIOTECH CORP.), GM-CSF, (IMMUNE CORP.), GMDP (PEPTECH LIMITED), Imiquimod (3M PHARMACEUTICALS), ImmTher (ENDOREX CORPORATION), ISCOMTM (ISCOTEC AB), Iscoprep 7.0. 3 TM (ISCOTEC AB), Loxoribine, LT-Oral Adjuvant (E. coli labile enterotoxin, protoxin, BERNA PRODUCTS CORP.), MTP-PE (CIBA-GEIGY LTD), Murametide, (VACSYN S. A.), Murapalmitine (VACSYN S. A.), Pluronic L121 (IDEC PHARMACEUTICALS CORP.), PMMA (INSTITUT FUR PHARMAZEUTISCHE TECHNOLOGIE), SAF-1 (SYNTEX ADJUVANT FORMULATION CHIRON), Stearyl tyrosine (BIOCHEM THERAPEUTIC INC.), Theramidea (IMMUNO THERAPEUTICS INC.), Threonyl- MDP (CHIRON), FREUNDS complete adjuvant, FREUNDS incomplete adjuvant, aluminum hydroxide, dimethyldioctadecyl-ammonium bromide, Adjuvax (ALPHA- BETA TECHNOLOGY), Inject Alum (PIERCE), Monophosphoryl Lipid A (RIBI IMMUNOCHEM RESEARCH), MPL+TDM (RIBI IMMUNOCHEM RESEARCH), Titermax (CYTRX), QS21, t Ribi Adjuvant System, TiterMaxGold, QS21, Adjumer, Calcitrol, CTB, LT (E. coli toxin), LPS (lipopolysaccharide), Avridine, the CpG sequences (Singh et al., 1999 Singh, M. and Hagum, D., Nature Biotechnology 1999 17: 1075-81) toxins, toxoids, glycoproteins, lipids, glycolipids, bacterial cell walls, subunits (bacterial or viral), carbohydrate moieties (mono-, di , tri-, tetra-, oligo-and polysaccharide), various liposome formulations or saponins. Combinations of various adjuvants may be used with the antigen to prepare the immunogen formulations. Adjuvants administered parentally or for the induction of mucosal immunity may also be used.

The present invention also contemplates a vaccine composition useful to stimulate an immune response in a mammal characterized in that it comprises an antigenic peptide, or an antigenic cocktail composition.

The vaccine composition can be administered by various delivery methods including intravascularly, intraperitoneally, intramuscularly, intradermally, subcutaneously, orally, nasally or by inhalation. In an embodiment, the compositions can further include a pharmaceutically acceptable excipient and/or carrier.

"Administered" or "administering", as it applies in the present invention, refers to contact of a pharmaceutical, therapeutic, diagnostic agent or composition, to the subject, preferably a human.

The exact formulation of the vaccine composition will depend on the particular antigenic peptide, or an antigenic cocktail, or peptide- carrier conjugate, and the route of administration.

When employing more than one antigenic peptide, such two or more, or an antigenic cocktail composition, they may be used as a physical mixture or a fusion of two or more antigenic peptides to form molecular chimeras. The molecular chimeras may be produced, for example, by recombinant techniques, by the use of appropriate linkers for fusing previously prepared antigenic peptides or by co-linearly synthesizing the chimera with or without linkers such as, for example, PEG (poly ethylene glycol).

Also encompassed in the present invention is an antibody characterized in that it recognizes the antigenic peptide of the invention or the antigenic cocktail composition of the invention.

As used herein, an "antibody" is a protein molecule that reacts with a specific antigen and belongs to one or five distinct classes based on structural properties: IgA, IgD, IgE, IgG and IgM. The antibody can be monoclonal or polyclonal.

The antibodies of the invention can also be produced for use in passive immunotherapy, for use as diagnostic reagents, and for use as reagents in other processes such as affinity chromatography.

When used in passive immunotherapy, the antibody of the invention can be included in a pharmaceutical composition and administered to a mammal. In an embodiment, the pharmaceutical composition includes a pharmaceutically acceptable carrier, and optionally can include pharmaceutically acceptable excipients. The pharmaceutical composition can be administered intravascularly, intraperitoneally, intramuscularly, intradermally, subcutaneously, orally, nasally or by aerosol inhalation. Preferably, the pharmaceutical composition is administered intravascularly, intramuscularly, orally, nasally or by aerosol inhalation.

In an embodiment, the present invention includes an antibody, particularly a monoclonal antibodies, directed to the antigenic peptide or the antigenic cocktail composition of the invention. In particular, hybridomas can be generated using a peptide of SEQ ID NOS: 1-156 and recombinant derivative antibodies can be made using these hybridomas according to well-known genetic engineering methods (Winter, G.and Milstein C., Nature, 1991,349 293-299).

Other methods known in the art to humanize an antibody or produce an humanized antibody can be utilized as well. These methods can include but are not limited to the xenomouse technology developed by ABGENIX INC. (See, U.S. Patent Nos. 6,075, 181 and 6,150, 584) and the methods developed by BIOVATION, BIOINVENT INTERNATIONAL AB, PROTEIN DESIGN LABS, APPLIED MOLECULAR EVOLUTION, INC. , IMMGENICS PHARMACEUTICALS INC., MEDAREX INC. , CAMBRIDGE ANTIBODY TECHNOLOGY, ELAN, EOS BIOTECHNOLOGY, MEDIMMUNE, MORPHOSYS, UROGENSYS INC. , AVANIR PHARMACEUTICAL/XENEREX BIOSCIENCES, AFFIBODY AB, ALLEXION ANTIBODY TECHNOLOGIES, ARIUS RESEARCH INC., CELL TECH, XOMA, IDEC PHARMACEUTICALS, NEUGENESIS, EPICYTE, SEMBIOSYS GENETICS INC., BIOPROTEIN, GENZYME THERAPEUTICS, KIRIN, GEMINI SCIENCES, HEMATECH.

Likewise, other methods known in the art to screen human antibody secreting cells to SEQ ID NOS: 1-131 may be also be utilized.

As used herein, the term "humanized antibody" or other like terms means an antibody that includes a human protein sequence in at least a portion thereof. The amount of human protein sequence can vary depending on how the antibody is made.

A "fully humanized antibody" or "human antibody" as the terms or like terms are used herein can be made, for example, with xenomouse technology as discussed above or transforming human B cells with Epstein Barr virus (Traggiai et al, 2004 in Nat. Med. 10(8): 871-5). Other methods such as phage display techniques are also possible (Bradbury AR, Marks JD, 2004 in J. immunol. Methods, 290 (1-2): 29-49).

When recombinant techniques are employed to prepare an antigenic peptide in accordance with the present invention, nucleic acid molecules or fragments thereof encoding the polypeptides are preferably used.

Therefore the present invention also relates to a purified and isolated nucleic acid sequence comprising
i) a nucleotide sequence encoding an antigenic peptide of the invention,
ii) a nucleic acid sequence complementary to i),
iii) a degenerated nucleic acid sequence of i) or ii),
iv) a nucleic acid sequence capable of hybridizing under stringent conditions to i), ii) or iii),
v) a nucleic acid sequence encoding a truncation or an analog of an antigenic peptide of the invention,
vi) and/or a fragment of i), ii), iii), iv) or v).

"A purified and isolated nucleic acid sequence" refers to the state in which the nucleic acid molecule encoding the antigenic peptide the invention, or nucleic acid encoding such the antigenic peptide will be, in accordance with the present invention. Nucleic acid will be free or substantially free of material with which it is naturally associated such as other polypeptides or nucleic acids with which it is found in its natural environment, or the environment in which it is prepared (e. g. cell culture) when such preparation is by recombinant nucleic acid technology practised *in vitro* or *in vivo.*

The term "nucleic acid" is intended to refer either to DNA or to RNA.

In case the nucleic acid is DNA, then DNA which can be used herein is any polydeoxynuclotide sequence, including, e.g. double-stranded DNA, single-stranded DNA, double-stranded DNA wherein one or both strands are composed of two or more fragments, double-stranded DNA wherein one or both strands have an uninterrupted phosphodiester backbone, DNA containing one or more single-stranded portion(s) and one or more double-stranded portion(s), double-stranded DNA wherein the DNA strands are fully complementary, double-stranded DNA wherein the DNA strands are only partially complementary, circular DNA, covalently- closed DNA, linear DNA, covalently cross-linked DNA, cDNA, chemically- synthesized DNA, semi-synthetic DNA, biosynthetic DNA, naturally-isolated DNA, enzyme-digested DNA, sheared DNA, labeled DNA, such as radiolabeled DNA and fluorochrome-labeled DNA, DNA containing one or more non-naturally occurring species of nucleic acid.

DNA sequences that encode the antigenic peptide of the invention, or a fragment thereof, can be synthesized by standard chemical techniques, for example, the phosphotriester method or via automated synthesis methods and PCR methods.

The purified and isolated DNA sequence encoding the antigenic peptide according to the invention may also be produced by enzymatic techniques. Thus, restriction enzymes, which cleave nucleic acid molecules at predefined recognition sequences can be used to isolate nucleic acid sequences from larger nucleic acid molecules containing the nucleic acid sequence, such as DNA (or RNA) that codes for the antigenic peptide of the invention or for a fragment thereof.

Encompassed by the present invention is also a nucleic acid in the form of a polyribonucleotide (RNA), including, e.g., single-stranded RNA, double- stranded RNA, double-stranded RNA wherein one or both strands are composed of two or more fragments, double-stranded RNA wherein one or both strands have an uninterrupted phosphodiester backbone, RNA containing one or more single-stranded portion(s) and one or more double-stranded portion(s), double-stranded RNA wherein the RNA strands are fully complementary, double-stranded RNA wherein the RNA strands are only partially complementary, covalently crosslinked RNA, enzyme-digested RNA, sheared RNA, mRNA, chemically-synthesized RNA, semi-synthetic RNA, biosynthetic RNA, naturally-isolated RNA, labeled RNA, such as radiolabeled RNA and fluorochrome-labeled RNA, RNA containing one or more non-naturally- occurring species of nucleic acid.

The purified and isolated nucleic acid sequence, DNA or RNA, also comprises a purified and isolated nucleic acid sequence having substantial sequence identity or homology to a nucleic acid sequence encoding an antigenic peptide of the invention. Preferably, the nucleic acid will have substantial sequence identity for example at least 50%, 55%, 60%, 65%, 70%, 75%, 80%, or 85% nucleic acid identity; more preferably 90% nucleic acid identity; and most preferably at least 95%, 96%, 97%, 98%, or 99% sequence identity.

"Identity" as known in the art and used herein, is a relationship between two or more amino acid sequences or two or more nucleic acid sequences, as determined by comparing the sequences. It also refers to the degree of sequence relatedness between amino acid or nucleic acid sequences, as the case may be, as determined by the match between strings of such sequences. Identity and similarity are well known terms to skilled artisans and they can be calculated by conventional methods (for example see Computational Molecular Biology, Lesk, A. M. ed., Oxford University Press, New York, 1988; Biocomputing: Informatics and Genome Projects, Smith, D. W. ed., Academic Press, New York, 1993; Computer Analysis of Sequence Data, Part I, Griffin, A. M. and Griffin, H. G. eds., Humana Press, New Jersey, 1994; Sequence Analysis in Molecular Biology, von Heinje, G. Acadmeic Press, 1987; and Sequence Analysis Primer, Gribskov, M. and Devereux, J. eds. M. Stockton Press, New York, 1991, Carillo, H. and Lipman, D., SIAM J. Applied Math. 48:1073, 1988).
Methods which are designed to give the largest match between the sequences are generally preferred. Methods to determine identity and similarity are codified in publicly available computer programs including the GCG program package (Devereux J. et al., Nucleic Acids Research 12(1): 387, 1984); BLASTP, BLASTN, and FASTA (Atschul, S. F. et al. J. Molec. Biol. 215: 403-410, 1990). The BLAST X program is publicly available from NCBI and other sources (BLAST Manual, Altschul, S. et al. NCBI NLM NIH Bethesda, Md. 20894; Altschul, S. et al. J. Mol. Biol. 215: 403-410, 1990).

Also encompassed by the present invention is a nucleic acid sequence complementary to the antigenic peptide of the invention.

Also within the scope of the invention is a degenerated nucleic acid sequence having a sequence which differs from a nucleic acid sequence encoding the antigenic peptide of the invention, or a complementary sequence thereof, due to degeneracy in the genetic code. Such nucleic acid encodes functionally equivalent to the antigenic peptide of the invention but differs in sequence from the sequence due to degeneracy in the genetic code. This may result in silent mutations which do not affect the amino acid sequence. Any and all such nucleic acid variations are within the scope of the invention.

In addition, also considered is a nucleic acid sequence capable of hybridizing under stringent conditions, preferably high stringency conditions, to a nucleic acid sequence encoding the antigenic peptide of the invention, a nucleic acid sequence complementary thereof or a degenerated nucleic acid sequence thereof. Appropriate stringency conditions which promote DNA hybridization are known to those skilled in the art, or can be found in Current Protocols in Molecular Biology, John Wiley & Sons, N.Y. (1989), 6.3.1-6.3.6. For example, 6.0X sodium chloride/sodium citrate (SSC) at about 45°C, followed by a wash of 2.0XSSC at 50°C may be employed. The stringency may be selected based on the conditions used in the wash step. By way of example, the salt concentration in the wash step can be selected from a high stringency of about 0.2XSSC at 50°C. In addition, the temperature in the wash step can be at high stringency conditions, at about 65° C.

The present invention also includes a purified and isolated nucleic acid encoding an antigenic peptide of the invention comprising a nucleic acid sequence encoding a truncation or an analog of the antigenic peptide.

The invention also encompasses allelic variants of the disclosed purified and isolated nucleic sequence; that is, naturally-occurring alternative forms of the isolated and purified nucleic acid that also encode antigenic peptides that are identical, homologous or related to that encoded by the purified and isolated nucleic sequences. Alternatively, non-naturally occurring variants may be produced by mutagenesis techniques or by direct synthesis.

A fragment of the disclosed purified and isolated nucleic sequence is also considered and refers to a sequence containing less nucleotides in length than the nucleic acid sequence encoding the antigenic peptide, a nucleic acid sequence complementary thereof or a degenerated nucleic acid sequence thereof. This sequence can be used as long as it exhibits the same properties as the native sequence from which it derives. Preferably this sequence contains less than 90%, preferably less than 60%, in particular less than 30% amino acids in length than the respective purified and isolated nucleic sequence of the antigenic peptide.

Yet another concern of the present invention is to provide an expression vector comprising at least one copy of the purified and isolated nucleic acid sequence encoding an antigenic peptide of the invention as described above.

The choice of an expression vector depends directly, as it is well known in the art, on the functional properties desired, e.g., antigenic peptide expression and the host cell to be transformed or transfected.

Additionally, the expression vector may further comprise a promoter operably linked to the purified and isolated nucleic acid sequence of the invention. This means that the linked isolated and purified nucleic acid sequence encoding the antigenic peptide of the present invention is under control of a suitable regulatory sequence which allows expression, i.e. transcription and translation of the inserted isolated and purified nucleic acid sequence.

As used herein, the term "promoter" designates any additional regulatory sequences as known in the art e.g. a promoter and/or an enhancer, polyadenylation sites and splice junctions usually employed for the expression of the polypeptide or may include additionally one or more separate targeting sequences and may optionally encode a selectable marker. Promoters which can be used provided that such promoters are compatible with the host cell are e.g promoters obtained from the genomes of viruses such as polyoma virus, adenovirus (such as Adenovirus 2), papilloma virus (such as bovine papilloma virus), avian sarcoma virus, cytomegalovirus (such as murine or human cytomegalovirus immediate early promoter), a retrovirus, hepatitis-B virus, and Simian Virus 40 (such as SV 40 early and late promoters) or promoters obtained from heterologous mammalian promoters, such as the actin promoter or an immunoglobulin promoter or heat shock promoters.

Enhancers which can be used are e.g. enhancer sequences known from mammalian genes (globin, elastase, albumin, a-fetoprotein, and insulin) or enhancer from a eukaryotic cell virus e.g. the SV40 enhancer, the cytomegalovirus early promoter enhancer, the polyoma, and adenovirus enhancers.

A wide variety of host/expression vector combinations may be employed in expressing the nucleic acid sequences of this invention. Useful expression vectors, for example, may consist of segments of chromosomal, non-chromosomal and synthetic DNA sequences. Suitable vectors include derivatives of SV40 and known bacterial plasmids, e. g., E. coli plasmids col E1, pCR1, pBR322, pMB9 and their derivatives, plasmids such as RP4; phage DNAs, e. g., the numerous derivatives of phage X, e. g., NM989, and other phage DNA, e. g., M13 and filamentous single stranded phage DNA; yeast plasmids such as the 2µ plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like.

Another concern of the present invention is to provide a host cell (eukaryotic or prokaryotic) comprising a purified and isolated nucleic acid sequence of the invention or an expression vector as described above.

Typically, this host cell has been transformed or transfected with a purified and isolated nucleic acid sequence of the invention or an expression vector described herein.

The term "cell transfected" or "cell transformed" or "transfected/transformed cell" means the cell into which the extracellular DNA has been introduced and thus harbours the extracellular DNA. The DNA might be introduced into the cell so that the nucleic acid is replicable either as a chromosomal integrant or as an extra chromosomal element.

Transformation or transfection of appropriate eukaryotic or prokaryotic host cells with an expression vector comprising a purified an isolated DNA sequence according to the invention is accomplished by well known methods that typically depend on the type of vector used. With regard to these methods, see *e.g.,* Maniatis et al. 1982, Molecular Cloning, A laboratory Manual, Cold Spring Harbor Laboratory and commercially available methods.

A wide variety of unicellular host cells are useful in expressing the nucleic acid sequences of this invention. These hosts may include well known eukaryotic and prokaryotic hosts, such as strains of E. coli, Pseudomonas, Bacillus, Streptomyces, fungi such as yeasts, and animal cells, such as CHO, YB/20, NSO, SP2/0, R1.1, B-W and L-M cells, African Green Monkey kidney cells (e.*g.,* COS 1, COS 7, BSC1, BSC40, and BMT10), insect cells *(e.g.,* Sf9), and human cells and plant cells in tissue culture. Preferably, the host cell is a bacterial cell, more preferably an E. coli cell.

The present invention is also directed to a vaccine composition for the treatment and/or prevention of malaria comprising at least the antigenic peptide of the invention, or the antigenic cocktail composition of the invention.

In a further aspect, the present invention is also directed to an acid nuclei vaccine composition for the treatment and/or prevention of malaria comprising at least a purified and isolated nucleic acid sequence of the invention, or an expression vector comprising at least one copy of the purified and isolated nucleic acid sequence of the invention, fragments thereof, molecular chimeras thereof, combinations thereof and/or variants thereof.

Yet another aspect of the present invention is a method of producing an antigenic peptide for the preparation of a vaccine composition against a pathogen, characterized in that it comprises the steps of:
a) identifying the presence of a sequence encoding at least one coiled coil region in the genome of said pathogen,
b) selecting said sequence encoding at least one coiled coil region,
c) synthesizing an antigenic peptide corresponding to the sequence containing the at least one coiled coil region selected in step c),
d) contacting the synthesized antigenic peptide with a serum sample from at least one donor living in pathogen endemic areas; and
e) determining whether at least one antibody of said serum sample binds to the synthesized antigenic peptide.

Identification of the presence of a sequence encoding at least one coiled coil region in the genome of said pathogen can be done using computer programs based on algorithms.

For example, Plasmodium falciparum 3D7 genome was used for the bioinformatics analysis (Gardner, 2002). Software named *pftools* and containing programs for sensitive generalized sequence profiles (Bucher et al., 1996) was used to search for the short α-helical coiled coil regions. The coiled coil profiles were constructed using an alignment of amino acid sequences corresponding to the known coiled coil domain. Two profiles containing four and five heptad repeats were used for the analysis. The coiled coil regions selected by this approach were also tested by the COILS program.

The selected α-helical coiled coil containing proteins were further tested on their possible surface location and GPI anchoring by using the following programs: identification of potential signal peptides (PSORT, http://www.psort.org/psortb/, SignalP, http://www.cbs.dtu.dk/services/SignaIP/), transmembrane spanning regions (TMPRED http://www.ch.embnet.org/software/TMPRED_form.html and TMHMM http://www.cbs.dtu.dk/services/TMHMM), and GPI-anchored proteins http://mendel.imp.univie.ac.at/sat/gpi/gpi_server.htm1.

The same computer program, or another one, will then compare the selected sequence to the transcriptome and the proteome databases of said pathogen in order to, for example, identify proteins in the asexual erythrocytic stages (www.PlasmoDB.org). If the coiled coil score of this sequence is high and if it is present in both the transcriptome and the proteome databases, it will be selected for subsequent peptide synthesis.

Peptide synthesis is performed according to techniques well known in the art such as solid-phase Fmoc chemistry. Usually the crude peptide is then purified by RP-HPLC or any other purification techniques, and analyzed by mass spectrometry (MALDI-TOF).

Synthetic peptide is then contacted with one serum, or a panel of sera, from individuals living in malaria endemic areas, and which have been infected by the pathogen, to assess if said serum/sera bind to the synthetized antigen, and thus underlying a positive antibody response. These responses will be further analysed for the presence of IgG1 and IgG3 subclasses, which have been shown, in case the pathogen is Plasmodium falciparum, to mediate protection by the reduction of Pf growth by an antibody dependant cytotoxic inhibition (ADCI) mechanism (Oeuvray et al, 1994). Biological activity of antibodies will be tested in ADCI and invasion inhibition assays (Okoyeh et al, 1999, Haynes et al, 2002) using peptide specific antibodies purified by affinity chromatography.

Usually the pathogen will be selected among the group comprising Mycobacterium tuberculosis, Influenza, Toxoplasma, Ebola virus, Streptococcus, Plasmodium falciparum, meningicoccus and staphyloccoccus

In case the pathogen is Plasmodium falciparum, the proteome and transcriptome data available in the literature (Betts, 2002, Florens et al, 2002, Bozdech et al, 2003) also allow for assessing whether these molecules are present in the erythrocytic stage.

The present invention also encompasses a diagnostic tool for determining the presence of an antigenic peptide or an antigenic cocktail composition of the invention in a sample comprising:
i) contacting the sample with an antibody directed to the antigenic peptide or the antigenic cocktail composition of the invention, and
ii) determining whether said antibody binds to a component of said sample.

The present invention further encompasses a diagnostic tool for determining the presence of antibodies directed to the antigenic peptide or to the antigenic cocktail composition of the invention in a sample comprising:
i) contacting said sample with the antigenic peptide or the antigenic cocktail composition of the invention, and
ii) determining whether antibodies bind to a component of said antigenic peptide or to said antigenic cocktail composition of the invention.

As used herein, "a sample" is an aliquot or a representative portion of a substance, material or population. For example, a sample may be a sample of blood, biological tissue, urine or feces. Preferably, the sample is blood.

As used herein, "a donor" is an individual person that lives in pathogen endemic areas and that has been, or is suspected to be, infected by said pathogen.

A "component" refers to any amino acid, nucleic acid, lipid or motif that is recognized by the antibody of the invention.

Also encompassed by the present invention is a protein that comprises at least one antigenic peptide deriving from Plasmodium species, said antigenic peptide comprising at least one coiled coil region.

Preferably the protein comprises sequences selected from the group comprising the amino acid sequences SEQ ID N°1 to SEQ ID N°131, fragments thereof, molecular chimeras thereof, combinations thereof and/or variants thereof. Most preferably the protein is PF14_0089 and comprises SEQ ID N° 38.

Usually the protein containing at least one antigenic peptide of the invention will be chemically synthesized and tested for recognition in ELISA assays. However, any other recombinant technique or recovery method such as expressing the protein in a host cell from a nucleotide sequence encoding said protein is also considered.

The invention also encompassed a vaccine composition useful to stimulate an immune response in mammal comprising a protein containing at least one antigenic peptide of the invention and the use thereof.

Further encompassed in the present invention is an antibody that recognizes a protein containing at least one antigenic peptide of the invention and its use in diagnostic tools.

Also encompassed is a purified and isolated nucleic acid sequence comprising
i) a nucleotide sequence encoding the protein of the invention,
ii) a nucleic acid sequence complementary to i),
iii) a degenerated nucleic acid sequence of i) or ii),
iv) a nucleic acid sequence capable of hybridizing under stringent conditions to i), ii) or iii),
v) a nucleic acid sequence encoding a truncation or an analog of the protein of the invention,
vi) and/or a fragment of i), ii), iii), iv) or v).

The present invention also relates to an expression vector comprising at least one copy of said purified and isolated nucleic acid sequence encoding the protein of the invention, and a host cell comprising either said purified and isolated nucleic acid sequence or said expression vector.

### EXAMPLES

### Example 1

### Materials

Chemicals and solvents used for the synthesis were purchased from Fluka (Buchs, Switzerland) and Novabiochem (Laufelfinger, Switzerland). Pooled human sera from Tanzania, individual adult sera from Burkina Faso and Tanzania and merozoite slides were obtained from Swiss Tropical Institute (STI, Basel, Switzerland). Sporozoite slides were obtained from Nijmegen (Holland).

### Peptide Synthesis

All the peptides were synthesized at the Department of Biochemistry (University of Lausanne, Switzerland) using solid-phase Fmoc chemistry using the Advanced ChemTech (Hatley St George, UK) AC T348 Omega multi channel synthesizer and the Applied Biosystem synthesizer 431 A, Foster City, CA). Protein PF14_0089 was synthesized from residue 47 to 186 since the 1-46 fragment was predicted to contain a leader and a trans-membrane sequence (plasmodb.org; September 2004). Crude peptides were purified by RP-HPLC (C₁₈ preparative column) and analyzed by mass spectrometry (MALDI-TOF; Applied Biosystem).

### ELISA

Microtitre 96-well plates (Maxisorp F96, Nunc, Denmark) were coated overnight (O/N) at 4°C with 50 µl/well of peptide at a concentration of 5 µg/ml. Plates were washed using phosphate buffered saline solution containing 0.05% Tween 20 (PBS-T; Sigma, St-Louis, MO, USA) and saturated with 5% non-fat dry milk in PBS-T for 1 hour at room temperature (RT). Serial dilutions of individual mice sera or individual human sera diluted at 1/200 in PBS-T-2.5% non-fat dry milk (PBS-T-milk) were added into the plates and incubated for 1 hour at RT. After washing, goat anti-mouse or human polyvalent immunoglobulin conjugated to alkaline phosphatase (Sigma, St-Louis, MO, USA) diluted (1/1000) in PBS-T-milk was added and the plates incubated for 1 hour. Plates were washed and the presence of enzyme evidenced by the p-nitrophenylphosphate substrate (Sigma, St-Louis, MO, USA). Absorbance was measured at 405 nm with a Multiskan Ascent Reader. The test sample titre was determined as the highest dilution above the mean optical density (OD) value + 3 standard deviation (SD) of the negative control (normal mouse serum or human serum from naïve donors).

### Example 2: Specific Antibody Purification by Affinity Chromatography

### Antigen-Sepharose conjugate preparation

5 mg of antigen was dissolved in 1 mL of coupling buffer (0.1 M NaHCO₃ containing 0.5 M NaCl, pH 8.0). The CNBr-sepharose 4B (Amersham Bioscience AB, Uppsala, Sweden) was swollen in 1 mM HCl then the gel was washed with coupling buffer. The antigen solution was added to the gel and the mixture is stirred 1h at RT. After the coupling reaction, excess of antigen was washed away with coupling buffer. The remaining activated groups were blocked by treatment with ethanolamine (0.25 M; pH 8.0) for 30 min at RT. The gel was then washed with sodium acetate buffer (0.1 M; pH 4.0), followed by coupling buffer. The antigen-sepharose conjugate was either used or stored at 4°C in PBS (1x) containing 1 mM azide.

### Isolation of specific antibody

Pooled human sera was diluted four times with PBS (1x) containing 0.5 M sodium chloride and mixed with antigen-sepharose conjugate. This mixture was then stirred gently on a wheel O/N at 4°C. After centrifugation human sera were collected and stored at -20°C until further used. The antigen-sepharose conjugate was then washed with 5 mL of trizma base TRIS (20 mM containing 0.5 M NaCl, pH 8.0) then with 5 mL of TRIS (20 mM, pH 8.0). The elution of bound antibody was achieved with glycine (0.1 M, pH 2.5). The fractions obtained were neutralized with TRIS (1 M, pH 8).

### Indirect Fluorescence Antibody Test (IFAT)

Slides coated with Pf sporozoites were dried at RT for 30 minutes, fixed with 100% acetone at 4°C for 10 minutes, washed 2 times in PBS-T, dried carefully and blocked with 20 µL/well of PBS 5% fetal calf serum (FCS) for 30 minutes. Slides coated with *Pf* merozoites were fixed with 100% acetone at -20°C for 15 minutes and dried O/N at RT. The appropriate antibody or serum dilutions prepared in PBS 1x-2.5% non-fat dry milk, were distributed (10 µL/well) and incubated 1h at RT (sporozoite) or 30 minutes at 37°C (merozoite) in a humid chamber. After washings with PBS-T, goat anti-mouse polyvalent immunoglobulin conjugated with Alexa fluor 488 (Molecular Probes, Oregon, USA) diluted 1/50 in Evans blue solution (1/50000) was added (400µL/slide) and incubated 50 minutes at RT (sporozoite) or 30 minutes at 37°C (merozoite) in a humid chamber in the dark. After washings slides are covered with 50 % glycerol, sealed and read using Zeica 200 microscope.

### Mice immunizations

Five- to seven-weeks old female CB6F1 mice were purchased from Harlan (Horst, Holland). Mice were injected subcutaneously at the base of the tail with 20 µg of peptide dissolved in PBS (1x) or water and emulsified in Montanide ISA720. For the mix of different peptides or the fragments of Ma16P1.37 and for the fragments of PfB0145c and PfD0110w, 10 and 5 µg/mouse respectively were injected. Animals received a booster dose of antigen after 3 and 6 weeks. Ten days after each boost, mice were bled to assess the presence of specific antibody by ELISA. If positive IFAT is performed, monoclonal antibodies are isolated according to Winter and Milstein (Nature, 1991,349 293-299).

### Example 3:

### Results

The screening of the *Pf* genome using sequence profiles identified several proteins containing putative α-helical coiled coil regions. The regions which are present in the proteome and in the transcriptome databases and have the highest coiled coil scores have been selected for subsequent peptide synthesis. Through proteome and transcriptome data available in the literature (Betts, 2002, Florens et al, 2002, Bozdech et al, 2003) Applicants have assessed if these molecules are present in the erythrocytic stage. The combined analysis/assessment identified 131 coiled-coil regions. Thirty eight coiled-coil segments, 30-40 amino acids long with the highest coiled-coil score, present either in the same protein or in different ones were selected. All selected antigens were recognized at various degrees (5-60%) by a panel of sera from donors living in endemic areas (Table 2).

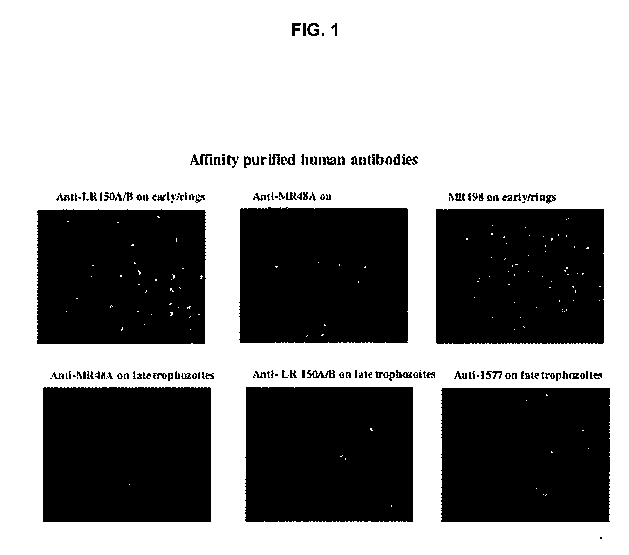

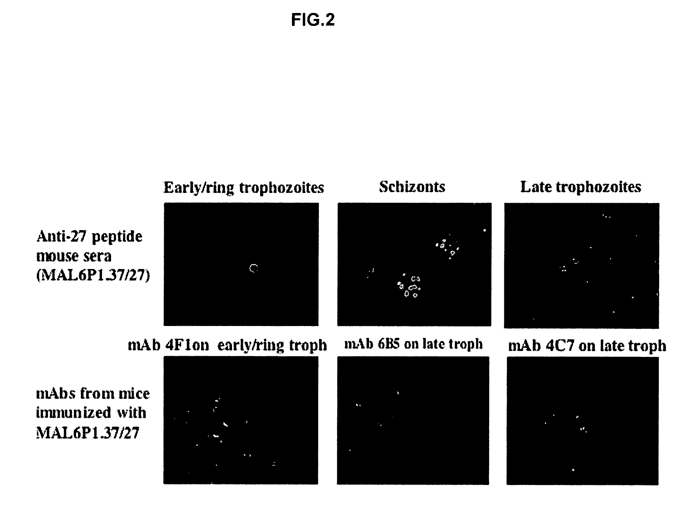

Thus, 17 new proteins were identified with length varying from 200 to 10,000 amino acids. Affinity chromatography purified antibodies specific for a number of peptides were also obtained from a pool of sera from adult donors living in Tanzania (Table 2). These antibodies are staining infected red blood cells (Fig. 1). In addition, ne of these proteins (PF14_0089) was chemically synthesized and tested for recognition in ELISA assays (Table 2). This novel protein is recognized by 78-100% of adult sera from Burkina Faso and Tanzania. Affinity purified antibodies recognize parasite infected red blood cells. In addition, this protein has also been associated with protection in Tanzanian children. they Peptides are also immunogenic in mice and specific sera are positive in i IFAT (Figure 2; Table 3).

Monoclonal antibodies, which are positive in IFAT, were also isolated (Figure 2; Table 3).

**Table 3**

| Immunogenicity of e selected fragments in CB6F1 mice. The Ab titers were determined by ELISA after the 3^{rd} immunisation; The immunofluorescence assay (IFAT) were done on different stage of the parasite. The results are expressed as following as negative (-); weak positive (+/-); positive (+) and not done (nd). | | | | | | |
|---|---|---|---|---|---|---|
| | **Mice immunisations with Protein / peptide** | | **IFAT** | | | **MonoAb** |
| **Groups** | | **Ab Titers** | ***Pf*Sporozoites** | **Early/ring trophozoites** | **Mid/late trophozoites** | |
| 1 | PFA0170c/1 | 100-218700 | - | +/- | - | - |
| | PFA0170c/1 | 100-72900 | | | | |
| 2 | PFB0145c/2 | 100 | - | + | + | |
| | PFB0145c/3 | 100-900 | | | | |
| 3 | PF14_0045/35 | 100-24 300 | - | +/- | - | |
| 4 | MAL6P1.37/27 | 100-24 300 | - | + | + | + (6B5, 4F1 and 4C7) |
| 5 | PF14_0089/38 | 100-218 700 | - | - | - | |
| 6 | PFB0145c/4 | 100-72900 | - | - | - | |
| 7 | MAL13P1.336/34 | 100 | - | - | - | |
| | PFD0110w/16 | 100 | | | | |
| | PF13_0277/33 | 100-900 | | | | |
| 8 | PF14_0089/38 | 100-72 900 | - | - | In progress | |
| | PFB0145c/2 | 100-900 | | | | |
| 9 | PFC0245c/14 | 100-24 300 | nd | - | - | |
| 10 | PF14_0175/37 | 100-2700 | nd | - | - | |
| | PF14_0045/35 | 100-900 | | | | |
| | MAL6P1.37/27 | 100-24 300 | | | | |
| | PFB0145c/4 | 100-2 700 | | | | |
| 11 | MAL13P1.336/34 | 100-24 300 | nd | - | In progress | |
| | PFC0245c/14 | 100-24 300 | | | | |
| | PF14_0175/37 | 100-900 | | | | |
| 12 | PF14_0089/39 | 100-218700 | nd | | + | |

### Example 4:

### Construction of a molecular chimera.

A molecular chimera construct containing peptides MR194, AS155.4, LR140 and AS155.9 was co-linearly synthesized using the Fmoc chemistry and the Applied Biosystem synthesizer 431 A. To avoid formation of neo-antigenic peptides, individual antigenic peptides were linked via the immunological silent linker poly-ethylen-glycol (PEG 01-63-0141; NovaBiochem/Merck) as follows: MR194-PEG-AS155.4-PEG-LR140-PEG-AS155.9.

This molecular chimera, called LR162A, is recognized in ELISA by 97% and 93% of sera form Burkina Faso and Tanzania, respectively.

### Example 5:

### Bio-informatics Screening

The Plasmodium falciparum 3D7 genome was used for the bioinformatics analysis (Gardner, 2002). Software named *pftools* and containing programs for sensitive generalized sequence profiles (Bucher et al., 1996) was used to search for the short α-helical coiled coil regions. The coiled coil profiles were constructed using an alignment of amino acid sequences corresponding to the known coiled coil region. Two profiles containing four and five heptad repeats were used for the analysis. The coiled coil regions selected by this approach were also tested by the COILS program.

The selected α-helical coiled coil containing proteins were further tested on their possible surface location and GPI anchoring by using the following programs: identification of potential signal peptides (PSORT, http://www.psort.org/psortb/, SignalP, http://www.cbs.dtu.dk/services/SignalP/). transmembrane spanning regions (TMPRED http://www.ch.embnet.org/software/TMPRED_form.html and TMHMM http://www.cbs.dtu.dk/services/TMHMM), and GPI-anchored proteins http://mendel.imp.univie.ac.at/sat/gpi/gpi server.html. Furthermore, the presence of the identified proteins in the asexual erythrocytic stages was also checked using the published data on the transcriptome and proteome of this stage of development of *P. falciparum* (www.PlasmoDB.org).

## Claims

1. An antigenic peptide deriving from Plasmodium species comprising at least one coiled coil region, **characterized in that** said antigenic peptide is selected from the group comprising the amino acid sequences SEQ ID N°1 to SEQ ID N°131, fragments thereof, molecular chimeras thereof, combinations thereof and/or variants thereof.

2. An antigenic cocktail composition deriving from Plasmodium species comprising at least 2 antigenic peptides according to claim 1 or mixtures thereof.

3. The use of the antigenic peptide of claim 1 or of the antigenic cocktail composition of claim 2 in the preparation of a vaccine composition for the stimulation of an immune response in a mammal.

4. A vaccine composition useful to stimulate an immune response in a mammal **characterized in that** it comprises the antigenic peptide of claim 1 or an antigenic cocktail composition of claim 2.

5. An antibody **characterized in that** it recognizes the antigenic peptide of claim 1 or the antigenic cocktail composition of claim 2.

6. A purified and isolated nucleic acid sequence comprising
vii) a nucleotide sequence encoding the antigenic peptide of claim 1,
viii) a nucleic acid sequence complementary to i),
ix) a degenerated nucleic acid sequence of i) or ii),
x) a nucleic acid sequence capable of hybridizing under stringent conditions to i), ii) or iii),
xi) a nucleic acid sequence encoding a truncation or an analog of the antigenic peptide of claim 1,
xii) and/or a fragment of i), ii), iii), iv) or v).

7. An expression vector comprising at least one copy of the purified and isolated nucleic acid sequence of claim 6.

8. A host cell comprising a purified and isolated nucleic acid sequence of claim 6 or an expression vector of claim 7.

9. Use of the vaccine composition of claim 4, in the manufacture of a medicament for the treatment and/or prevention of malaria.

10. A method of producing an antigenic peptide for the preparation of a vaccine composition against a pathogen, **characterized in that** it comprises the steps of:
a) identifying the presence of a sequence encoding at least one coiled coil region in the genome of said pathogen,
b) selecting said sequence encoding at least one coiled coil region,
c) synthesizing an antigenic peptide corresponding to the sequence containing the at least one coiled coil region selected in step b),
d) contacting the synthesized antigenic peptide with a serum sample from at least one donor living in pathogen endemic areas; and
e) determining whether at least one antibody of said serum sample binds to the synthesized antigenic peptide.

11. A diagnostic tool for determining the presence of the antigenic peptide of claim 1 or an antigenic cocktail composition of claim 2 in a sample comprising:
i) contacting the sample with an antibody directed to the antigenic peptide of claim 1 or to the antigenic cocktail composition of claim 2, and
ii) determining whether said antibody binds to a component of said sample.

12. A diagnostic tool for determining the presence of antibodies to peptide of claim 1 or an antigenic cocktail composition of claim 2 in a sample comprising:
i) contacting said sample with the antigenic peptide of claim 1 or the antigenic cocktail composition of claim 2, and
ii) determining whether antibodies bind to a component of said antigenic peptide of claim 1 or to said antigenic cocktail composition of claim 2.

13. A protein **characterized in that** it comprises an antigenic peptide of claim 1.

14. The protein of claim 13 **characterized in that** it is protein PF14_0089
